# EUROPEAN PATENT APPLICATION

(11) **EP 4 601 407 A1**
(43) Date of publication of application: **13.08.2025**
(21) Application number: 25156155.1
(22) Date of filing: 06.02.2025
(51) Int. Cl.: H04W 84/18, G01N 33/00, G08B 21/12

(54) **SYSTEMS, APPARATUSES, METHODS, AND COMPUTER PROGRAM PRODUCTS FOR OUTPUTTING AN ADJUSTED MULTIPOINT LOCATION GAS INDICATION AGGREGATION INTERFACE COMPONENT AND A POINT LOCATION ALERT GAS INTERFACE COMPONENT TO A MULTIPOINT LOCATION GAS INDICATION STATUS INTERFACE**

(30) Priority: 09.02.2024 US 202418437780
(71) Applicant: Life Safety Distribution GmbH, 1180 Rolle (CH)
(72) Inventor: NING, Qin, Charlotte, 28202 (US); TAO, Chen, Charlotte, 28202 (US); WANGUO, Shang, Charlotte, 28202 (US); YANLONG, Sun, Charlotte, 28202 (US); YU, Wei, Charlotte, 28202 (US); XINYUE, Liu, Charlotte, 28202 (US)
(74) Representative: Haseltine Lake Kempner LLP

(57) **Abstract**

Apparatus, methods, and computer program products for outputting interface components for rendering to the multipoint location gas indication status interface in a multipoint location gas analysis system. A method may include accessing multipoint location gas indication data, generating a multipoint location gas indication aggregation interface component based at least in part on the multipoint location gas indication data, outputting the multipoint location gas indication aggregation interface component for rendering to a multipoint location gas indication status interface of a computing device associated with the multipoint location gas indication aggregation interface component, generating a point location alert gas interface component based at least in part on point location alert gas data, generating an adjusted multipoint location gas indication aggregation interface component, outputting the point location alert gas interface component and the adjusted multipoint location gas indication aggregation interface component for rendering to the multipoint location gas indication status interface.

## Description

### TECHNOLOGICAL FIELD

Embodiments of the present disclosure relate generally to outputting an adjusted multipoint location gas indication aggregation interface component and a point location alert gas interface component to a multipoint location gas indication status interface.

### BACKGROUND

Applicant has identified many technical challenges and difficulties associated with outputting an adjusted multipoint location gas indication aggregation interface component and a point location alert gas interface component to a multipoint location gas indication status interface. Through applied effort, ingenuity, and innovation, Applicant has solved problems related to outputting an adjusted multipoint location gas indication aggregation interface component and a point location alert gas interface component to a multipoint location gas indication status interface by developing solutions embodied in the present disclosure, which are described in detail below.

### BRIEF SUMMARY

Various embodiments described herein relate to outputting an adjusted multipoint location gas indication aggregation interface component and a point location alert gas interface component to a multipoint location gas indication status interface.

In accordance with one aspect of the disclosure an example apparatus is provided. In some embodiments, the apparatus may comprise at least one processor and at least one memory including program code. In some embodiments, the at least one memory and the program code are configured to, with the at least one processor, cause the apparatus to access multipoint location gas indication data. In some embodiments, the at least one memory and the program code are configured to, with the at least one processor, cause the apparatus to generate a multipoint location gas indication aggregation interface component based at least in part on the multipoint location gas indication data. In some embodiments, the at least one memory and the program code are configured to, with the at least one processor, cause the apparatus to output the multipoint location gas indication aggregation interface component for rendering to a multipoint location gas indication status interface of a computing device associated with the multipoint location gas indication aggregation interface component. In some embodiments, the at least one memory and the program code are configured to, with the at least one processor, cause the apparatus to generate a point location alert gas interface component based at least in part on point location alert gas data. In some embodiments, the at least one memory and the program code are configured to, with the at least one processor, cause the apparatus to generate an adjusted multipoint location gas indication aggregation interface component. In some embodiments, the at least one memory and the program code are configured to, with the at least one processor, cause the apparatus to output the point location alert gas interface component and the adjusted multipoint location gas indication aggregation interface component for rendering to the multipoint location gas indication status interface of the computing device associated with the multipoint location gas indication aggregation interface component.

In some embodiments, the multipoint location gas indication status interface comprises a first multipoint location gas indication status portion and a second multipoint location gas indication status portion.

In some embodiments, the multipoint location gas indication aggregation interface component is configured to be rendered to the first multipoint location gas indication status portion and the second multipoint location gas indication status portion of the multipoint location gas indication status interface.

In some embodiments, the adjusted multipoint location gas indication aggregation interface component is configured to be rendered to the first multipoint location gas indication status portion of the multipoint location gas indication status interface and the point location alert gas interface component to be rendered to the second multipoint location gas indication status portion of the multipoint location gas indication status interface.

In some embodiments, the at least one memory and the program code are configured to, with the at least one processor, cause the apparatus to generate a point location service status interface component, a point location maintenance fault interface component, a point location instrument fault interface component, or a point location event interface component.

In some embodiments, the at least one memory and the program code are configured to, with the at least one processor, cause the apparatus to generate the point location alert gas data. In some embodiments, generating the multipoint location gas indication data comprises determining that a point location site is associated with a gas anomaly event using an optical analysis technique.

In some embodiments, the multipoint location gas indication data is indicative of at least one of a plurality of point location site identifiers, a plurality of point location unique identification identifiers, a plurality of point location gas type identifier, a plurality of point location gas concentration indicators, or a plurality of point location status indicators.

In some embodiments, the multipoint location gas indication aggregation interface component comprises a plurality of point location gas indication interface components.

In some embodiments, each of the plurality of point location gas indication interface components is configured to display at least one of a point location site interface component, a point location unique identification interface component, a point location gas type interface component, a point location gas concentration interface component, or a point location status interface component.

In some embodiments, at least a portion of the point location alert gas interface component is associated with a deuteranopia type configuration, a protanopia type configuration, a tritanopia type configuration, or a monochromacy type configuration.

In some embodiments, the point location alert gas interface component is configured to display at least one of a point location alert gas subsidiary interface component or a point location alert action gas interface component.

In accordance with another aspect of the disclosure an example method is provided. In some embodiments, the method may include accessing multipoint location gas indication data. In some embodiments, the method may include generating a multipoint location gas indication aggregation interface component based at least in part on the multipoint location gas indication data. In some embodiments, the method may include outputting the multipoint location gas indication aggregation interface component for rendering to a multipoint location gas indication status interface of a computing device associated with the multipoint location gas indication aggregation interface component. In some embodiments, the method may include generating a point location alert gas interface component based at least in part on point location alert gas data. In some embodiments, the method may include generating an adjusted multipoint location gas indication aggregation interface component. In some embodiments, the method may include outputting the point location alert gas interface component and the adjusted multipoint location gas indication aggregation interface component for rendering to the multipoint location gas indication status interface of the computing device associated with the multipoint location gas indication aggregation interface component.

In some embodiments, the multipoint location gas indication status interface comprises a first multipoint location gas indication status portion and a second multipoint location gas indication status portion.

In some embodiments, the multipoint location gas indication aggregation interface component is configured to be rendered to the first multipoint location gas indication status portion and the second multipoint location gas indication status portion of the multipoint location gas indication status interface.

In some embodiments, the adjusted multipoint location gas indication aggregation interface component is configured to be rendered to the first multipoint location gas indication status portion of the multipoint location gas indication status interface and the point location alert gas interface component to be rendered to the second multipoint location gas indication status portion of the multipoint location gas indication status interface.

In some embodiments, the method may include generating a point location service status interface component, a point location maintenance fault interface component, a point location instrument fault interface component, or a point location event interface component.

In some embodiments, the method may include generating the point location alert gas data. In some embodiments, generating the multipoint location gas indication data comprises determining that a point location site is associated with a gas anomaly event using an optical analysis technique.

In some embodiments, the multipoint location gas indication data is indicative of at least one of a plurality of point location site identifiers, a plurality of point location unique identification identifiers, a plurality of point location gas type identifier, a plurality of point location gas concentration indicators, or a plurality of point location status indicators.

In some embodiments, the multipoint location gas indication aggregation interface component comprises a plurality of point location gas indication interface components.

In some embodiments, each of the plurality of point location gas indication interface components is configured to display at least one of a point location site interface component, a point location unique identification interface component, a point location gas type interface component, a point location gas concentration interface component, or a point location status interface component.

In some embodiments, at least a portion of the point location alert gas interface component is associated with a deuteranopia type configuration, a protanopia type configuration, a tritanopia type configuration, or a monochromacy type configuration.

In some embodiments, the point location alert gas interface component is configured to display at least one of a point location alert gas subsidiary interface component or a point location alert action gas interface component.

In accordance with another aspect of the disclosure an example computer program product is provided. In some embodiments, the computer program product comprises at least one non-transitory computer-readable storage medium having computer-readable program code portions stored therein, the computer-readable program code portions comprising an executable portion.

In some embodiments, the computer-readable program code portions comprising an executable portion are configured to access multipoint location gas indication data. In some embodiments, the computer-readable program code portions comprising an executable portion are configured to generate a multipoint location gas indication aggregation interface component based at least in part on the multipoint location gas indication data. In some embodiments, the computer-readable program code portions comprising an executable portion are configured to output the multipoint location gas indication aggregation interface component for rendering to a multipoint location gas indication status interface of a computing device associated with the multipoint location gas indication aggregation interface component. In some embodiments, the computer-readable program code portions comprising an executable portion are configured to generate a point location alert gas interface component based at least in part on point location alert gas data. In some embodiments, the computer-readable program code portions comprising an executable portion are configured to generate an adjusted multipoint location gas indication aggregation interface component. In some embodiments, the computer-readable program code portions comprising an executable portion are configured to output the point location alert gas interface component and the adjusted multipoint location gas indication aggregation interface component for rendering to the multipoint location gas indication status interface of the computing device associated with the multipoint location gas indication aggregation interface component.

In some embodiments, the multipoint location gas indication status interface comprises a first multipoint location gas indication status portion and a second multipoint location gas indication status portion.

In some embodiments, the multipoint location gas indication aggregation interface component is configured to be rendered to the first multipoint location gas indication status portion and the second multipoint location gas indication status portion of the multipoint location gas indication status interface.

In some embodiments, the adjusted multipoint location gas indication aggregation interface component is configured to be rendered to the first multipoint location gas indication status portion of the multipoint location gas indication status interface and the point location alert gas interface component to be rendered to the second multipoint location gas indication status portion of the multipoint location gas indication status interface.

In some embodiments, the computer-readable program code portions comprising an executable portion are configured to generate a point location service status interface component, a point location maintenance fault interface component, a point location instrument fault interface component, or a point location event interface component.

In some embodiments, the computer-readable program code portions comprising an executable portion are configured to generate the point location alert gas data. In some embodiments, generating the multipoint location gas indication data comprises determining that a point location site is associated with a gas anomaly event using an optical analysis technique.

In some embodiments, the multipoint location gas indication data is indicative of at least one of a plurality of point location site identifiers, a plurality of point location unique identification identifiers, a plurality of point location gas type identifier, a plurality of point location gas concentration indicators, or a plurality of point location status indicators.

In some embodiments, the multipoint location gas indication aggregation interface component comprises a plurality of point location gas indication interface components.

In some embodiments, each of the plurality of point location gas indication interface components is configured to display at least one of a point location site interface component, a point location unique identification interface component, a point location gas type interface component, a point location gas concentration interface component, or a point location status interface component.

In some embodiments, at least a portion of the point location alert gas interface component is associated with a deuteranopia type configuration, a protanopia type configuration, a tritanopia type configuration, or a monochromacy type configuration.

In some embodiments, the point location alert gas interface component is configured to display at least one of a point location alert gas subsidiary interface component or a point location alert action gas interface component.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms above, non-limiting and non-exhaustive embodiments of the subject disclosure will now be described with reference to the accompanying drawings which are not necessarily drawn to scale. The components illustrated in the accompanying drawings may or may not be present in certain embodiments described herein. Some embodiments may include fewer (or more) components than those shown in the figures in accordance with an example embodiment of the present disclosure.
FIG. 1 illustrates example multipoint location gas analysis system configured to communicate with various client devices in accordance with various example embodiments of the present disclosure;
FIG. 2 depicts a schematic block diagram of example circuitry to perform various operations in accordance with various example embodiments of the present disclosure;
FIG. 3 illustrates an example multipoint location gas indication aggregation interface component in accordance with various example embodiments of the present disclosure;
FIG. 4 illustrates an example multipoint location gas indication status interface in accordance with various example embodiments of the present disclosure;
FIG. 5 illustrates an example multipoint location gas indication status interface in accordance with various example embodiments of the present disclosure;
FIG. 6 illustrates an example multipoint location gas indication status interface in accordance with various example embodiments of the present disclosure;
FIG. 7 illustrates an example point location alert gas interface component in accordance with various example embodiments of the present disclosure;
FIG. 8 illustrates an example adjusted multipoint location gas indication aggregation interface component in accordance with various example embodiments of the present disclosure;
FIG. 9 illustrates an example multipoint location gas indication status interface in accordance with various example embodiments of the present disclosure;
FIG. 10 illustrates an example point location service status interface component in accordance with various example embodiments of the present disclosure;
FIG. 11 illustrates an example point location maintenance fault interface component in accordance with various example embodiments of the present disclosure;
FIG. 12 illustrates an example point location instrument fault interface component in accordance with various example embodiments of the present disclosure;
FIG. 13 illustrates an example point location event interface component in accordance with various example embodiments of the present disclosure; and
FIG. 14 depicts a flowchart diagram illustrating example operations for outputting an adjusted multipoint location gas indication aggregation interface component and a point location alert gas interface component to a multipoint location gas indication status interface in accordance with an example embodiment of the present disclosure.

### DETAILED DESCRIPTION

Example embodiments now will be more fully described with reference to the accompanying drawings, wherein like reference numerals are used to refer to like elements throughout. In the following description, for purposes of explanation, numerous specific details are set forth in order to provide a thorough understanding of the various embodiments. It is evident, however, that the various embodiments can be practiced without these specific details. It should be understood that some, but not all embodiments of the present disclosure are shown and described herein. Indeed, embodiments of the disclosure may be embodied in many different forms, and accordingly this disclosure should not be construed as limited to the embodiments set forth herein; rather, these embodiments are provided so that this disclosure will satisfy applicable legal requirements.

### Overview

Example embodiments disclosed herein address technical problems associated with outputting an adjusted multipoint location gas indication aggregation interface component and a point location alert gas interface component to a multipoint location gas indication status interface using a multipoint location gas analysis system. As would be understood by one skilled in the field to which this disclosure pertains, there are numerous example scenarios in which a user may desire outputting an adjusted multipoint location gas indication aggregation interface component and a point location alert gas interface component to a multipoint location gas indication status interface using a multipoint location gas analysis system.

In many applications, it is often desirable to monitor various gas parameters at a particular point. For example, in a semiconductor manufacturing process it may be desirable to monitor gas parameters such as gas type, gas concentration, and gas status at a particular point associated with the semiconductor manufacturing process (e.g., proximate a machine that performs one or more subprocesses in a semiconductor manufacturing process).

Example solutions for monitoring various gas parameters at a particular point include placing a gas analysis system proximate the particular point where it is desirable to monitor the gas parameters and outputting a particular monitored gas parameter for the particular point to a user interface. However, such example solutions are inefficient and costly. For example, many example gas analysis systems are only able to monitor individual gas parameters at individual points. This increases costs and reduces efficiency because multiple gas analysis systems must be deployed at each particular point in order to monitor various gas parameters at multiple particular points. As another example, many gas analysis systems are unable to output multiple interface components to a single interface with at least one interface component conveying particular points having acceptable monitored gas parameters (e.g., a monitored gas concentration threshold is below an acceptable threshold) and at least one other interface component conveying particular points having unacceptable monitored gas parameters (e.g., a monitored gas concentration threshold is above an acceptable threshold), which reduces efficiency and increases costs.

Thus, to address these and/or other issues related to outputting an adjusted multipoint location gas indication aggregation interface component and a point location alert gas interface component to a multipoint location gas indication status interface using a multipoint location gas analysis system, example systems, apparatuses, and/or methods for outputting an adjusted multipoint location gas indication aggregation interface component and a point location alert gas interface component to a multipoint location gas indication status interface using a multipoint location gas analysis system are disclosed herein. For example, an embodiment in this disclosure, described in greater detail below, includes accessing multipoint location gas indication data, generating a multipoint location gas indication aggregation interface component based at least in part on the multipoint location gas indication data, outputting the multipoint location gas indication aggregation interface component for rendering to a multipoint location gas indication status interface of a computing device associated with the multipoint location gas indication aggregation interface component, generating a point location alert gas interface component based at least in part on point location alert gas data, generating an adjusted multipoint location gas indication aggregation interface component, and outputting the point location alert gas interface component and the adjusted multipoint location gas indication aggregation interface component for rendering to the multipoint location gas indication status interface of the computing device associated with the multipoint location gas indication aggregation interface component. Thus, embodiments disclosed herein enable for monitoring of multiple different gas parameters at multiple different points in an efficient and cost effective manner.

### Definitions

The following explanations of terms are provided to better describe the present disclosure and to guide those of ordinary skill in the art in the practice of the present disclosure.

As used herein, the term "multipoint location gas analysis system" refers to a hardware platform, software platform, computing device, and/or the like configured to support and/or maintain a gas analysis system, interfaces associated with a gas analysis system, and/or interface components associated with a gas analysis system. For example, a multipoint location gas analysis system may comprise a VertexVC4 system and/or components associated with a VertexVC4 system configured to support and/or maintain interfaces and/or interface components associated with a multipoint location gas analysis system. For example, a multipoint location gas analysis system may be configured to generate, manage, output, and/or update multipoint location gas indication status interfaces, multipoint location gas indication aggregation interface components, adjusted multipoint location gas indication aggregation interface components, point location alert gas interface components, point location service status interface components, point location maintenance fault interface components, point location instrument fault interface components, point location event interface components, and/or the like. As another example, a multipoint location gas analysis system is configured to identify, determine, generate, and/or output multipoint location gas indication data and/or point location alert gas data. As another example, a multipoint location gas analysis system is configured to identify, determine, detect, and/or output a gas anomaly event using an optical analysis technique.

As used herein, the term "multipoint location gas analysis data repository" refers to a location, such as a database stored on a memory device, which is accessible by one or more computing devices for retrieval and storage of data associated with a multipoint location gas analysis system. For example, a multipoint location gas analysis data repository may include multipoint location gas indication data, point location alert gas data, and/or the like. A multipoint location gas analysis data repository may be a dedicated device and/or a part of a larger repository. A multipoint location gas analysis data repository may be dynamically updated or be static. In some embodiments, a multipoint location gas analysis data repository is encrypted in order to limit unauthorized access of data associated with a multipoint location gas analysis system.

As used herein used herein, the terms "multipoint location gas analysis application" or "multipoint location gas analysis app" refer to a dedicated software program, application, platform, service, web browser, or computer-executable application software programmed or configured to run on a client device and/or a multipoint location gas analysis system which provides a user access to the multipoint location gas analysis system and its associated functionality. In some embodiments, the multipoint location gas analysis application may include hardware, software, or combinations thereof operating remotely (e.g., on a server). In some embodiments, a multipoint location gas analysis application is designed to execute on mobile devices, such as tablets or smartphones. For example, in certain embodiments, an app is provided that executes on mobile device operating systems such as iOS^{®}, Android^{®}, or Windows^{®}. These platforms typically provide frameworks that allow apps to communicate with one another and with particular hardware and software components of mobile devices. For example, the mobile operating systems named above each provide frameworks for interacting with location services circuitry, wired and wireless network interfaces, user contacts, and other applications. Communication with hardware and software modules executing outside of the app is typically provided via application programming interfaces (APIs) provided by the mobile device operating system. In some embodiments, a multipoint location gas analysis application is designed to execute on a multipoint location gas analysis system.

As used herein, the term "multipoint location gas indication status interface" refers to a graphical user interface of a multipoint location gas analysis system that is configured to enable one or more users to view and engage with one or more multipoint location gas analysis system, views, and/or interface components. In some embodiments, for example, a multipoint location gas indication status interface is configured to enable one or more users to view and engage with one or more of a multipoint location gas indication aggregation interface component, an adjusted multipoint location gas indication aggregation interface component, a point location alert gas interface component, a point location service status interface component, a point location maintenance fault interface component, a point location instrument fault interface component, and/or a point location event interface component. In some embodiments, a multipoint location gas indication status interface may be rendered to a client device based on data provided by the multipoint location gas analysis system. In some embodiments, such data and instructions are facilitated by a dedicated software application running on the client device. In other embodiments, such data and instructions are provided through a web browser running on the client device. In some embodiments, a multipoint location gas indication status interface may be rendered to a user interface associated with a multipoint location gas analysis system. In some embodiments, such data and instructions are facilitated by a dedicated software application running on the multipoint location gas analysis system. In other embodiments, such data and instructions are provided through a web browser running on the multipoint location gas analysis system. In some embodiments, a multipoint location gas indication status interface may comprise a first multipoint location gas indication status portion and/or a second multipoint location gas indication status portion.

As used herein, the term "first multipoint location gas indication status portion" refers to a portion of a multipoint location gas indication status interface. In some embodiments, a portion of a multipoint location gas indication aggregation interface component may be configured to be rendered to a first multipoint location gas indication status portion. In some embodiments, an adjusted multipoint location gas indication aggregation interface component may be configured to be rendered to a first multipoint location gas indication status portion.

As used herein, the term "second multipoint location gas indication status portion" refers to a portion of a multipoint location gas indication status interface. In some embodiments, a portion of a multipoint location gas indication aggregation interface component may be configured to be rendered to a second multipoint location gas indication status portion. In some embodiments, a point location alert gas interface component may be configured to be rendered to a second multipoint location gas indication status portion. In some embodiments, a second multipoint location gas indication status portion may occupy a different portion of a multipoint location gas indication status interface than a first multipoint location gas indication status portion.

As used herein, the term "multipoint location gas indication aggregation interface component" refers to a graphical user interface element that is rendered to, or as a portion of, a multipoint location gas indication status interface. In some embodiments, a multipoint location gas indication aggregation interface component may be configured to display a plurality of point location gas indication interface components. For example, a multipoint location gas indication aggregation interface component may be configured to display four point location gas indication interface components. In some embodiments, a multipoint location gas indication aggregation interface component may be based at least in part on multipoint location gas indication data.

In some embodiments, a multipoint location gas indication aggregation interface component may be configured to be rendered to a first multipoint location gas indication status portion of a multipoint location gas indication status interface and a second multipoint location gas indication status portion of a multipoint location gas indication status interface. Said differently, a multipoint location gas indication aggregation interface component may be configured such that at least a portion of a multipoint location gas indication aggregation interface component is rendered on a first multipoint location gas indication status portion and at least another portion of a multipoint location gas indication aggregation interface component is rendered on a second multipoint location gas indication status portion.

As used herein, the term "multipoint location gas indication data" refers to one or more items of data indicative of a plurality of point location site identifiers, a plurality of point location unique identification identifiers, a plurality of point location gas concentration indicators, a plurality of point location gas type identifiers, and/or a plurality of point location status indicators.

As used herein, the term "point location site identifier" refers to one or more items of data configured to identify a physical location from which one or more gases are collected and/or identified such that an analysis can be performed on the one or more gases by a multipoint location gas analysis system. In this regard, a point location site identifier may comprise text string(s), numerical character(s), alphabetical character(s), alphanumeric code(s), American Standard Code for Information Interchange (ASCII) character(s), encryption keys, identification certificates, a pointer, an Internet Protocol (IP) address, a MAC address, a URL, a memory address, other unique identifier, or a combination thereof configured to identify a physical location from which one or more gases are collected and/or identified such that an analysis can be performed on the one or more gases by a multipoint location gas analysis system. For example, a point location site identifier may be Site A Floor Five, Site A Floor Two, Site B Floor Three, and/or the like.

In some embodiments, a point location site identified by a point location site identifier may be in fluid communication with a multipoint location gas analysis system. In this regard, for example, a pipe, tube, connector, any other connection means, and/or the like may provide fluid communication between a point location site identified by a point location site identifier and a multipoint location gas analysis system. For example, a pipe, tube, connector, any other connection means, and/or the like may provide fluid communication between a point location site identified by a point location site identifier as Site B Floor Three and a multipoint location gas analysis system located at Site B Floor Two. In this regard, a multipoint location gas analysis system may be configured to perform analysis on one or more gases present at one or more particular physical locations.

As used herein, the term "point location unique identification identifier" refers to one or more items of data by which a point location site may be uniquely identified within a multipoint location gas analysis system. For example, a point location unique identification identifier may comprise text string(s), numerical character(s), alphabetical character(s), alphanumeric code(s), American Standard Code for Information Interchange (ASCII) character(s), encryption keys, identification certificates, a pointer, an Internet Protocol (IP) address, a MAC address, a URL, a memory address, other unique identifier, or a combination thereof configured to uniquely identify a point location site within a multipoint location gas analysis system.

As used herein, the term "point location gas type identifier" refers to one or more items of data by which a type of gas associated with a point location site may be identified within a multipoint location gas analysis system (e.g., a type of gas at a point location site as determined by a multipoint location gas analysis system). For example, a point location gas type identifier may comprise text string(s), numerical character(s), alphabetical character(s), alphanumeric code(s), American Standard Code for Information Interchange (ASCII) character(s), encryption keys, identification certificates, a pointer, an Internet Protocol (IP) address, a MAC address, a URL, a memory address, other unique identifier, or a combination thereof configured to identify a point location gas type associated with a point location site within a multipoint location gas analysis system.

In some embodiments, a point location gas type identifier may be configured to identify any type of gas that may be analyzed by a multipoint location gas analysis system. In some embodiments, a point location gas type identifier may be configured to identify a type of gas associated with a semiconductor manufacturing process when a multipoint location gas analysis system is configured to analyze gases associated with the semiconductor manufacturing process. For example, a point location gas type identifier may be configured to identify one or more of arsine (AsH3), Diborane (B2H6), germane (GeH4), and/or the like.

As used herein, the term "point location gas concentration indicator" refers to one or more items of data by which a concentration of a gas associated with a point location site may be indicated within a multipoint location gas analysis system (e.g., a concentration of a gas at a point location site as determined by a multipoint location gas analysis system). For example, a point location gas concentration indicator may comprise text string(s), numerical character(s), alphabetical character(s), alphanumeric code(s), American Standard Code for Information Interchange (ASCII) character(s), encryption keys, identification certificates, a pointer, an Internet Protocol (IP) address, a MAC address, a URL, a memory address, other unique identifier, or a combination thereof configured to indicate a gas concentration of a gas associated with a point location site within a multipoint location gas analysis system. In some embodiments, for example, a point location gas concentration indicator may be configured to indicate a gas concentration 0.000 parts per million (ppm) of a gas associated with a point location site. As another example, a point location gas concentration indicator may be configured to indicate a gas concentration of 1.137 ppm of associated with a point location site.

As used herein, the term "point location status indicator" refers to one or more items of data by which a status of a point location site may be indicated within a multipoint location gas analysis system (e.g., a status associated with a gas at a point location site as determined by a multipoint location gas analysis system). For example, a point location status indicator may comprise text string(s), numerical character(s), alphabetical character(s), alphanumeric code(s), American Standard Code for Information Interchange (ASCII) character(s), encryption keys, identification certificates, a pointer, an Internet Protocol (IP) address, a MAC address, a URL, a memory address, other unique identifier, or a combination thereof configured to indicate a status of a gas associated with a point location site. In some embodiments, for example, a point location status indicator may be configured to indicate that a concentration of a gas associated with a point location site is at an acceptable level or an unacceptable level. As another example, a point location status indicator may be configured to indicate that a type of gas associated with a point location site is acceptable or unacceptable.

As used herein, the term "point location gas indication interface component" refers to a graphical user interface element that is rendered to, or as a portion of, a multipoint location gas indication aggregation interface component. In some embodiments, a point location gas indication interface component may be rendered proximate one or more other point location gas indication interface components on a multipoint location gas indication aggregation interface component. For example, a point location gas indication interface component may be rendered proximate three other point location gas indication interface components (e.g., a multipoint location gas indication aggregation interface component may be configured to display four point location gas indication interface components).

In some embodiments, a point location gas indication interface component may be based at least in part on multipoint location gas indication data. In some embodiments, a point location gas indication interface component may be configured to display one or more of a point location site interface component, a point location unique identification interface component, a point location gas type interface component, point location gas concentration interface component, and/or a point location status indicator interface component.

As used herein, the term "point location site interface component" refers to a graphical user interface element that is rendered to, or as a portion of, a point location gas indication interface component. In some embodiments, a point location site interface component may be based at least in part on a point location site identifier. In this regard, a point location site interface component may be configured to display text, color, graphics, and/or the like representative of a point location site identifier. For example, a point location site interface component may be configured to display text, color, graphics, and/or the like based on a point location site identifier associated with a point location site of Site A Floor Five (e.g., text comprising Site A Floor Five).

In some embodiments, a point location site interface component may be configured to be updated. For example, if a point location site identifier associated with a point location site interface component is updated, the point location site interface component may be configured to display text, color, graphics, and/or the like representative of the updated point location site identifier (e.g., updated text comprising Site A Floor Four). In some embodiments, a point location site interface component may be configured to be updated in real-time or near real-time.

As used herein, the term "point location unique identification interface component" refers to a graphical user interface element that is rendered to, or as a portion of, a point location gas indication interface component. In some embodiments, a point location unique identification interface component may be based at least in part on a point location unique identification identifier. In this regard, a point location unique identification interface component may be configured to display text, color, graphics, and/or the like representative of a point location unique identification identifier. For example, a point location unique identification interface component may be configured to display text, color, graphics, and/or the like based on a point location unique identification identifier associated with a point location unique identification of 123223 (e.g., text comprising 123223).

In some embodiments, a point location unique identification interface component may be configured to be updated. For example, if a point location unique identification identifier associated with a point location unique identification interface component is updated, the point location unique identification interface component may be configured to display text, color, graphics, and/or the like representative of the updated point location unique identification identifier (e.g., updated text comprising 322321). In some embodiments, a point location unique identification interface component may be configured to be updated in real-time or near real-time.

As used herein, the term "point location gas type interface component" refers to a graphical user interface element that is rendered to, or as a portion of, a point location gas indication interface component. In some embodiments, a point location gas type interface component may be based at least in part on a point location gas type identifier. In this regard, a point location gas type interface component may be configured to display text, color, graphics, and/or the like representative of a point location gas type identifier. For example, a point location gas type interface component may be configured to display text, color, graphics, and/or the like based on a point location gas type identifier associated with a point location gas type of arsine (AsH3) (e.g., text comprising AsH3).

In some embodiments, a point location gas type interface component may be configured to be updated. For example, if a point location gas type identifier associated with a point location gas type interface component is updated, the point location gas type interface component may be configured to display text, color, graphics, and/or the like representative of the updated point location gas type identifier (e.g., updated text comprising B2H6). In some embodiments, a point location gas type interface component may be configured to be updated in real-time or near real-time.

As used herein, the term "point location gas concentration interface component" refers to a graphical user interface element that is rendered to, or as a portion of, a point location gas indication interface component. In some embodiments, a point location gas concentration interface component may be based at least in part on a point location gas concentration indicator. In this regard, a point location gas concentration interface component may be configured to display text, color, graphics, and/or the like representative of a point location gas concentration indicator. For example, a point location gas concentration interface component may be configured to display text, color, graphics, and/or the like based on a point location gas concentration indicator associated with a point location gas concentration of 0.000 ppm (e.g., text comprising 0.000 ppm).

In some embodiments, a point location gas concentration interface component may be configured to be updated. For example, if a point location gas concentration indicator associated with a point location gas concentration interface component is updated, the point location gas concentration interface component may be configured to display text, color, graphics, and/or the like representative of the updated point location gas concentration indicator (e.g., updated text comprising 1.137 ppm). In some embodiments, a point location gas concentration interface component may be configured to be updated in real-time or near real-time.

As used herein, the term "point location status interface component" refers to a graphical user interface element that is rendered to, or as a portion of, a point location gas indication interface component. In some embodiments, a point location status interface component may be based at least in part on a point location status indicator. In this regard, a point location status interface component may be configured to display text, color, graphics, and/or the like representative of a point location status indicator. For example, a point location status interface component may be configured to display text, color, graphics, and/or the like based on a point location status indicator associated with a point location status of an acceptable gas concentration level (e.g., text stating that there is an acceptable gas concentration level, color (e.g., green) indicating that there is an acceptable gas concentration level, symbol(s) (e.g., a check mark) indicating that there is an acceptable gas concentration level).

In some embodiments, a point location status interface component may be configured to be updated. For example, if a point location status indicator associated with a point location status interface component is updated, the point location status interface component may be configured to display text, color, graphics, and/or the like representative of the updated point location status indicator (e.g., updated text stating that there is an unacceptable gas concentration level, updated color (e.g., red) indicating that there is an unacceptable gas concentration level, updated symbol(s) (e.g., a warning symbol) indicating that there is an unacceptable gas concentration level). In some embodiments, a point location status interface component may be configured to be updated in real-time or near real-time.

As used herein, the term "adjusted multipoint location gas indication aggregation interface component" refers to a graphical user interface element that is rendered to, or as a portion of, a multipoint location gas indication status interface. In some embodiments, an adjusted multipoint location gas indication aggregation interface component may be configured to display a plurality of point location gas indication interface components. For example, an adjusted multipoint location gas indication aggregation interface component may be configured to display four point location gas indication interface components. In some embodiments, a multipoint location gas indication aggregation interface component may be based at least in part on multipoint location gas indication data and/or point location alert gas data.

In some embodiments, an adjusted multipoint location gas indication aggregation interface component may be configured to be rendered to a first multipoint location gas indication status portion of a multipoint location gas indication status interface. In this regard, an adjusted multipoint location gas indication aggregation interface component may occupy a smaller portion of a multipoint location gas indication status interface than a multipoint location gas indication aggregation interface component. In some embodiments, an adjusted multipoint location gas indication aggregation interface component may be rendered proximate a point location alert gas interface component on a multipoint location gas indication status interface. For example, an adjusted multipoint location gas indication aggregation interface component may be configured to be rendered to a first multipoint location gas indication status portion of a multipoint location gas indication status interface and a point location alert gas interface component may be configured to be rendered to a second multipoint location gas indication status portion of a multipoint location gas indication status interface.

As used herein, the term "point location alert gas data" refers to one or more items of data indicative of a point location site being associated with a gas anomaly event. In this regard, for example, a multipoint location gas analysis system may be configured to generate point location alert gas data by determining that a point location site is associated with a gas anomaly event. In some embodiments, a multipoint location gas analysis system may be configured to generate point location alert gas data by determining that a point location site is associated with a gas anomaly event using an optical analysis technique.

In some embodiments, a gas anomaly event may occur when a point location gas concentration associated with a point location site is greater than a point location gas concentration threshold. In this regard, for example, point location alert gas data may indicate that a point location gas concentration associated with a point location site is greater than a point location gas concentration threshold (e.g., a point location gas concentration associated with a point location site is 1.137 ppm and a point location gas concentration threshold is 0.500 ppm). In some embodiments, a gas anomaly event may occur when a point location gas type associated with a point location site is different than an expected point location gas type for the point location site (e.g., a type of gas is present at a point location site that should not be present at the point location site). In this regard, for example, point location alert gas data may indicate that a point location gas type associated with a point location site is different that an expected point location gas type for the point location site.

As used herein, the term "optical analysis technique" refers to one or more techniques, procedures, algorithms, processes, routines, and/or the like configured to be performed by a multipoint location gas analysis system to determine that a point location site is associated with a gas anomaly event. In some embodiments, an optical analysis technique may include a multipoint location gas analysis system performing optical analysis on a Chemcassette^{®} paper to determine if a point location site is associated with a gas anomaly event.

As used herein, the term "point location alert gas interface component" refers to refers to a graphical user interface element that is rendered to, or as a portion of, a multipoint location gas indication status interface. In some embodiments, a point location alert gas interface component may be configured to display one or more point location alert gas subsidiary interface components and/or one or more point location alert action gas interface components. In some embodiments, a point location alert gas interface component may be based at least in part on multipoint location gas indication data and/or point location alert gas data.

In some embodiments, a point location alert gas interface component may be configured to be rendered to a second multipoint location gas indication status portion of a multipoint location gas indication status interface. In some embodiments, a point location alert gas interface component may be rendered proximate an adjusted multipoint location gas indication aggregation interface component on a multipoint location gas indication status interface. For example, an adjusted multipoint location gas indication aggregation interface component may be configured to be rendered to a first multipoint location gas indication status portion of a multipoint location gas indication status interface and a point location alert action gas interface component may be configured to be rendered to a second multipoint location gas indication status portion of a multipoint location gas indication status interface.

As used herein, the term "point location alert gas subsidiary interface component" refers to a graphical user interface element that is rendered to, or as a portion of, a point location alert gas interface component. In some embodiments, a point location alert gas subsidiary interface component may be based at least in part on multipoint location gas indication data and/or point location alert gas data. In some embodiments, a point location alert gas subsidiary interface component may be configured to display text, colors, symbols, and/or the like representative of one or more of a point location site identifier, a point location unique identification identifier, a point location gas concentration indicator, a point location gas type identifier, and/or a point location status indicator associated with a point location site associated with a gas anomaly event. For example, for a point location site associated with a point location gas concentration greater than a point location gas concentration threshold, a point location alert gas subsidiary interface component may be configured to display text, colors, symbols, and/or the like representative of a point location gas concentration identifier associated with the point location site.

As used herein, the term "point location alert action gas interface component" refers to a graphical user interface element that is rendered to, or as a portion of, a point location alert gas interface component. In some embodiments, a point location alert action gas interface component may be configured to be selected by a user such that a user can select the point location alert action gas interface component to acknowledge that a gas anomaly event has occurred. In some embodiments, a point location alert action gas interface component may be configured to be selected by a user such that a user can trigger one or more actions in response to a gas anomaly event by selecting the point location alert action gas interface component. For example, the one or more actions that can be triggered in response to a gas anomaly event by a user selecting a point location alert action gas interface component may include triggering an alarm to inform individuals associated with the point location site of the gas anomaly event. As another example, the one or more actions that can be triggered in response to a gas anomaly event by a user selecting a point location alert action gas interface component may include causing an evacuation of the point location site. As another example, the one or more actions that can be triggered in response to a gas anomaly event by a user selecting a point location alert action gas interface component may include causing machinery and/or processes associated with the point location site to shut down.

As used herein, the term "deuteranopia type configuration" refers to a particular type of interface and/or interface component configuration. In some embodiments, an interface and/or interface component associated with a deuteranopia type configuration may be an interface and/or interface component configured such that the interface and/or interface component is accessible to a user affected by deuteranopia. For example, an interface and/or interface component associated with deuteranopia type configuration may be an interface and/or interface component configured to display particular text, colors (e.g., colors other than green), and/or symbols such that the interface and/or interface component is accessible to a user affected by deuteranopia.

In some embodiments, at least a portion of a multipoint location gas indication aggregation interface component, an adjusted multipoint location gas indication aggregation interface component, a point location alert gas interface component, a point location service status interface component, a point location maintenance fault interface component, a point location instrument fault interface component, a point location event interface component, and/or a point location gas indication interface component may be associated with a deuteranopia type configuration. In this regard, for example, at least a portion of a multipoint location gas indication aggregation interface component may be configured such that the multipoint location gas indication aggregation interface component is accessible to a user affected by deuteranopia. As another example, at least a portion of an adjusted multipoint location gas indication aggregation interface component may be configured such that the adjusted multipoint location gas indication aggregation interface component is accessible to a user affected by deuteranopia. As another example, at least a portion of a point location alert gas interface component may be configured such that the point location alert gas interface component is accessible to a user affected by deuteranopia. As another example, at least a portion of a point location service status interface component may be configured such that the point location service status interface component is accessible to a user affected by deuteranopia.

As another example, at least a portion of a point location maintenance fault interface component may be configured such that the point location maintenance fault interface component is accessible to a user affected by deuteranopia. As another example, at least a portion of a point location instrument fault interface component may be configured such that the point location instrument fault interface component is accessible to a user affected by deuteranopia. As another example, at least a portion of a point location event interface component may be configured such that the point location event interface component is accessible to a user affected by deuteranopia. As another example, at least a portion of a point location gas indication interface component may be configured such that the point location gas indication interface component is accessible to a user affected by deuteranopia.

As used herein, the term "protanopia type configuration" refers to a particular type of interface and/or interface component configuration. In some embodiments, an interface and/or interface component associated with a protanopia type configuration may be an interface and/or interface component configured such that the interface and/or interface component is accessible to a user affected by protanopia. For example, an interface and/or interface component associated with protanopia type configuration may be an interface and/or interface component configured to display particular text, colors (e.g., colors other than red), and/or symbols such that the interface and/or interface component is accessible to a user affected by protanopia.

In some embodiments, at least a portion of a multipoint location gas indication aggregation interface component, an adjusted multipoint location gas indication aggregation interface component, a point location alert gas interface component, a point location service status interface component, a point location maintenance fault interface component, a point location instrument fault interface component, a point location event interface component, and/or a point location gas indication interface component may be associated with a protanopia type configuration. In this regard, for example, at least a portion of a multipoint location gas indication aggregation interface component may be configured such that the multipoint location gas indication aggregation interface component is accessible to a user affected by protanopia. As another example, at least a portion of an adjusted multipoint location gas indication aggregation interface component may be configured such that the adjusted multipoint location gas indication aggregation interface component is accessible to a user affected by protanopia. As another example, at least a portion of a point location alert gas interface component may be configured such that the point location alert gas interface component is accessible to a user affected by protanopia. As another example, at least a portion of a point location service status interface component may be configured such that the point location service status interface component is accessible to a user affected by protanopia.

As another example, at least a portion of a point location maintenance fault interface component may be configured such that the point location maintenance fault interface component is accessible to a user affected by protanopia. As another example, at least a portion of a point location instrument fault interface component may be configured such that the point location instrument fault interface component is accessible to a user affected by protanopia. As another example, at least a portion of a point location event interface component may be configured such that the point location event interface component is accessible to a user affected by protanopia. As another example, at least a portion of a point location gas indication interface component may be configured such that the point location gas indication interface component is accessible to a user affected by protanopia.

As used herein, the term "tritanopia type configuration" refers to a particular type of interface and/or interface component configuration. In some embodiments, an interface and/or interface component associated with a tritanopia type configuration may be an interface and/or interface component configured such that the interface and/or interface component is accessible to a user affected by tritanopia. For example, an interface and/or interface component associated with tritanopia type configuration may be an interface and/or interface component configured to display particular text, colors, and/or symbols such that the interface and/or interface component is accessible to a user affected by tritanopia.

In some embodiments, at least a portion of a multipoint location gas indication aggregation interface component, an adjusted multipoint location gas indication aggregation interface component, a point location alert gas interface component, a point location service status interface component, a point location maintenance fault interface component, a point location instrument fault interface component, a point location event interface component, and/or a point location gas indication interface component may be associated with a tritanopia type configuration. In this regard, for example, at least a portion of a multipoint location gas indication aggregation interface component may be configured such that the multipoint location gas indication aggregation interface component is accessible to a user affected by tritanopia. As another example, at least a portion of an adjusted multipoint location gas indication aggregation interface component may be configured such that the adjusted multipoint location gas indication aggregation interface component is accessible to a user affected by tritanopia. As another example, at least a portion of a point location alert gas interface component may be configured such that the point location alert gas interface component is accessible to a user affected by tritanopia. As another example, at least a portion of a point location service status interface component may be configured such that the point location service status interface component is accessible to a user affected by tritanopia.

As another example, at least a portion of a point location maintenance fault interface component may be configured such that the point location maintenance fault interface component is accessible to a user affected by tritanopia. As another example, at least a portion of a point location instrument fault interface component may be configured such that the point location instrument fault interface component is accessible to a user affected by tritanopia. As another example, at least a portion of a point location event interface component may be configured such that the point location event interface component is accessible to a user affected by tritanopia. As another example, at least a portion of a point location gas indication interface component may be configured such that the point location gas indication interface component is accessible to a user affected by tritanopia.

As used herein, the term "monochromacy type configuration" refers to refers to a particular type of interface and/or interface component configuration. In some embodiments, an interface and/or interface component associated with a monochromacy type configuration may be an interface and/or interface component configured such that the interface and/or interface component is accessible to a user affected by monochromacy. For example, an interface and/or interface component associated with monochromacy type configuration may be an interface and/or interface component configured to display particular text, colors, and/or symbols such that the interface and/or interface component is accessible to a user affected by monochromacy.

In some embodiments, at least a portion of a multipoint location gas indication aggregation interface component, an adjusted multipoint location gas indication aggregation interface component, a point location alert gas interface component, a point location service status interface component, a point location maintenance fault interface component, a point location instrument fault interface component, a point location event interface component, and/or a point location gas indication interface component may be associated with a monochromacy type configuration. In this regard, for example, at least a portion of a multipoint location gas indication aggregation interface component may be configured such that the multipoint location gas indication aggregation interface component is accessible to a user affected by monochromacy. As another example, at least a portion of an adjusted multipoint location gas indication aggregation interface component may be configured such that the adjusted multipoint location gas indication aggregation interface component is accessible to a user affected by monochromacy. As another example, at least a portion of a point location alert gas interface component may be configured such that the point location alert gas interface component is accessible to a user affected by monochromacy. As another example, at least a portion of a point location service status interface component may be configured such that the point location service status interface component is accessible to a user affected by monochromacy.

As another example, at least a portion of a point location maintenance fault interface component may be configured such that the point location maintenance fault interface component is accessible to a user affected by monochromacy. As another example, at least a portion of a point location instrument fault interface component may be configured such that the point location instrument fault interface component is accessible to a user affected by monochromacy. As another example, at least a portion of a point location event interface component may be configured such that the point location event interface component is accessible to a user affected by monochromacy. As another example, at least a portion of a point location gas indication interface component may be configured such that the point location gas indication interface component is accessible to a user affected by monochromacy.

As used herein, the term "point location service status interface component" refers to a graphical user interface element that is rendered to, or as a portion of, a multipoint location gas indication status interface. In some embodiments, a point location service status interface component may be based at least in part on multipoint location gas indication data and/or point location alert gas data. In some embodiments, a point location service status interface component may be configured to display text, colors, symbols, and/or the like representative of one or more service statuses associated with a multipoint location gas analysis system. In some embodiments, for example, a point location service status interface component may be configured to display text, colors, symbols, and/or the like representative of an age of a Chemcassette^{®} paper associated with a multipoint location gas analysis system (e.g., 5 days). As another example, a point location service status interface component may be configured to display text, colors, symbols, and/or the like representative of an optic cleaning timeline associated with a multipoint location gas analysis system (e.g., 180 days). As another example, a point location service status interface component may be configured to display text, colors, symbols, and/or the like representative of a filter age associated with a multipoint location gas analysis system (e.g., 176 days).

In some embodiments, a point location service status interface component may be configured to be rendered to a second multipoint location gas indication status portion of a multipoint location gas indication status interface. In some embodiments, a point location service status interface component may be rendered proximate an adjusted multipoint location gas indication aggregation interface component on a multipoint location gas indication status interface. For example, an adjusted multipoint location gas indication aggregation interface component may be configured to be rendered to a first multipoint location gas indication status portion of a multipoint location gas indication status interface and a point location service status interface component may be configured to be rendered to a second multipoint location gas indication status portion of a multipoint location gas indication status interface.

As used herein, the term "point location maintenance fault interface component" refers to a graphical user interface element that is rendered to, or as a portion of, a multipoint location gas indication status interface. In some embodiments, a point location maintenance fault interface component may be based at least in part on multipoint location gas indication data and/or point location alert gas data. In some embodiments, a point location maintenance fault interface component may be configured to display text, colors, symbols, and/or the like representative of one or more maintenance faults associated with a multipoint location gas analysis system. For example, a point location maintenance fault interface component may be configured to display text, colors, symbols, and/or the like representative of a low flow rate maintenance fault.

In some embodiments, a point location maintenance fault interface component may be configured to be rendered to a second multipoint location gas indication status portion of a multipoint location gas indication status interface. In some embodiments, a point location maintenance fault interface component may be rendered proximate an adjusted multipoint location gas indication aggregation interface component on a multipoint location gas indication status interface. For example, an adjusted multipoint location gas indication aggregation interface component may be configured to be rendered to a first multipoint location gas indication status portion of a multipoint location gas indication status interface and a point location maintenance fault interface component may be configured to be rendered to a second multipoint location gas indication status portion of a multipoint location gas indication status interface.

As used herein, the term "point location instrument fault interface component" refers to a graphical user interface element that is rendered to, or as a portion of, a multipoint location gas indication status interface. In some embodiments, a point location instrument fault interface component may be based at least in part on multipoint location gas indication data and/or point location alert gas data. In some embodiments, a point location instrument fault interface component may be configured to display text, colors, symbols, and/or the like representative of one or more instrument faults associated with a multipoint location gas analysis system. For example, a point location instrument fault interface component may be configured to display text, colors, symbols, and/or the like representative of an optic noise instrument fault (e.g., noise associated with optics configured to perform an optical analysis technique).

In some embodiments, a point location instrument fault interface component may be configured to be rendered to a second multipoint location gas indication status portion of a multipoint location gas indication status interface. In some embodiments, a point location instrument fault interface component may be rendered proximate an adjusted multipoint location gas indication aggregation interface component on a multipoint location gas indication status interface. For example, an adjusted multipoint location gas indication aggregation interface component may be configured to be rendered to a first multipoint location gas indication status portion of a multipoint location gas indication status interface and a point location instrument fault interface component may be configured to be rendered to a second multipoint location gas indication status portion of a multipoint location gas indication status interface.

As used herein, the term "point location event interface component" refers to a graphical user interface element that is rendered to, or as a portion of, a multipoint location gas indication status interface. In some embodiments, a point location event interface component may be based at least in part on multipoint location gas indication data and/or point location alert gas data In some embodiments, a point location event interface component may be configured to display text, colors, symbols, and/or the like representative of one or more events associated with a multipoint location gas analysis system. For example, a point location event interface component may be configured to display text, colors, symbols, and/or the like representative of an event that comprises a gas anomaly event associated with the multipoint location gas analysis system. As another example, a point location event interface component may be configured to display text, colors, symbols, and/or the like representative of a maintenance fault associated with a multipoint location gas analysis system (e.g., a low flow rate maintenance fault).

In some embodiments, a point location event interface component may be configured to be rendered to a second multipoint location gas indication status portion of a multipoint location gas indication status interface. In some embodiments, a point location event interface component may be rendered proximate an adjusted multipoint location gas indication aggregation interface component on a multipoint location gas indication status interface. For example, an adjusted multipoint location gas indication aggregation interface component may be configured to be rendered to a first multipoint location gas indication status portion of a multipoint location gas indication status interface and a point location event interface component may be configured to be rendered to a second multipoint location gas indication status portion of a multipoint location gas indication status interface.

The terms "data," "content," "digital content," "digital content object," "signal," "information," and similar terms may be used interchangeably to refer to data capable of being transmitted, received, and/or stored in accordance with embodiments of the present disclosure. Thus, use of any such terms should not be taken to limit the spirit and scope of embodiments of the present disclosure. Further, where a computing device is described herein to receive data from another computing device, it will be appreciated that the data may be received directly from another computing device or may be received indirectly via one or more intermediary computing devices, such as, for example, one or more servers, relays, routers, network access points, base stations, hosts, and/or the like, sometimes referred to herein as a "network." Similarly, where a computing device is described herein to send data to another computing device, it will be appreciated that the data may be transmitted directly to another computing device or may be transmitted indirectly via one or more intermediary computing devices, such as, for example, one or more servers, relays, routers, network access points, base stations, hosts, and/or the like.

As used herein, the term "computer-readable storage medium" refers to a non-transitory, physical or tangible storage medium (e.g., volatile or non-volatile memory), which may be differentiated from a "computer-readable transmission medium," which refers to an electromagnetic signal. Such a medium can take many forms, including, but not limited to a non-transitory computer-readable storage medium (e.g., non-volatile media, volatile media), and transmission media. Transmission media include, for example, coaxial cables, copper wire, fiber optic cables, and carrier waves that travel through space without wires or cables, such as acoustic waves and electromagnetic waves, including radio, optical, infrared waves, or the like. Signals include man-made, or naturally occurring, transient variations in amplitude, frequency, phase, polarization or other physical properties transmitted through the transmission media. Examples of non-transitory computer-readable media include a magnetic computer readable medium (e.g., a floppy disk, hard disk, magnetic tape, any other magnetic medium), an optical computer readable medium (e.g., a compact disc read only memory (CD-ROM), a digital versatile disc (DVD), a Blu-Ray disc, or the like), a random access memory (RAM), a programmable read only memory (PROM), an erasable programmable read only memory (EPROM), a FLASH-EPROM, or any other non-transitory medium from which a computer can read. The term computer-readable storage medium is used herein to refer to any computer-readable medium except transmission media. However, it will be appreciated that where embodiments are described to use a computer-readable storage medium, other types of computer-readable mediums can be substituted for or used in addition to the computer-readable storage medium in alternative embodiments.

The terms "client device," "computing device," "network device," "computer," "user equipment," and similar terms may be used interchangeably to refer to computer hardware and/or software that is configured to access a service made available by a system (e.g., a multipoint location gas analysis system). The system is often (but not always) on another computer system, in which case the client device accesses the service by way of a network. Client devices may include, without limitation, smart phones, tablet computers, laptop computers, wearables, personal computers, enterprise computers, and the like.

As used herein, the term "circuitry" refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of "circuitry" applies to all uses of this term herein, including in any claims. As a further example, the term "circuitry" also includes an implementation comprising one or more processors and/or portion(s) thereof and accompanying software and/or firmware. As another example, the term "circuitry" as used herein also includes, for example, a baseband integrated circuit or applications processor integrated circuit for a mobile phone or a similar integrated circuit in a server, a cellular network device, other network device, and/or other computing device.

As used herein, the terms "application," "software application," "app," "product," "service" or similar terms refer to a computer program or group of computer programs designed to perform coordinated functions, tasks, or activities for the benefit of a user or group of users. A software application can run on a server or group of servers (e.g., a physical or virtual servers in a cloud-based computing environment). In certain embodiments, an application is designed for use by and interaction with one or more local, networked or remote computing devices, such as, but not limited to, client devices.

The terms "database," "resource," "repository," and/or similar terms used herein interchangeable may refer to a collection of records or data that is stored in a computer-readable storage medium using one or more database types. The term "database type" may refer to a type of database, such as a hierarchical database, network database, relational database (e.g., Aurora, RDS), entity-relationship database, object database (e.g., S3), document database, semantic database, graph database, noSqL database (e.g., DynamoDB), and/or the like.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

As used herein, the phrases "in one embodiment," "according to one embodiment," "in some embodiments," and the like generally refer to the fact that the particular feature, structure, or characteristic following the phrase may be included in at least one embodiment of the present disclosure. Thus, the particular feature, structure, or characteristic may be included in more than one embodiment of the present disclosure such that these phrases do not necessarily refer to the same embodiment.

As used herein, the terms "illustrative," "example," "exemplary" and the like are used to mean "serving as an example, instance, or illustration" with no indication of quality level. Any implementation described herein as "exemplary" or "example" is not necessarily to be construed as preferred or advantageous over other implementations.

The terms "about," "approximately," "generally," "substantially," or the like, when used with a number, may mean that specific number, or alternatively, a range in proximity to the specific number, as understood by persons of skill in the art field and may be used to refer to within manufacturing and/or engineering design tolerances for the corresponding materials and/or elements as would be understood by the person of ordinary skill in the art, unless otherwise indicated.

If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

If the specification presents a list, unless stated otherwise, it is to be understood that each individual element of that list, and every combination of components of that list, is a separate embodiment. For example, "1, 2, 3, 4, and 5" encompasses, among numerous embodiments, 1; 2; 3; 1 and 2; 3 and 5; 1, 3, and 5; and 1, 2, 4, and 5.

The term "plurality" refers to two or more items.

The term "set" refers to a collection of one or more items.

The term "or" is used herein in both the alternative and conjunctive sense, unless otherwise indicated.

### Example Systems and Apparatuses of the Disclosure

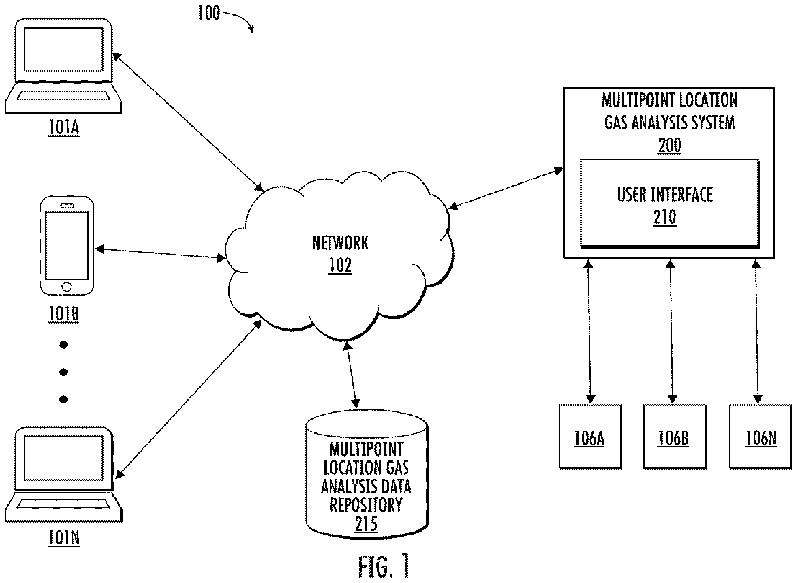

Referring now to FIG. 1, an example environment 100 within which some embodiments of the present disclosure operate is illustrated. The example environment 100 comprises one or more point location sites 106A-106N, a multipoint location gas analysis system 200, a multipoint location gas analysis data repository 215, and/or a plurality of client devices 101A-101N. In some embodiments, the multipoint location gas analysis system 200, the multipoint location gas analysis data repository 215, and/or the plurality of client devices 101A-101N may be communicatively connected through a network 102. Accordingly, user(s) may access the multipoint location gas analysis system 200 and/or the multipoint location gas analysis data repository 215 via the network 102 using one or more of client devices 101A-101N. In some embodiments, the multipoint location gas analysis system 200 may be in communication with at least one repository, such as a multipoint location gas analysis data repository 215. Such repository(ies) may be hosted by the multipoint location gas analysis system 200 (e.g., the multipoint location gas analysis data repository 215 may be a component of the multipoint location gas analysis system 200) or otherwise hosted by devices in communication with the multipoint location gas analysis system 200 (e.g., the multipoint location gas analysis data repository 215 may be a separate from the multipoint location gas analysis system 200 and in communication with the multipoint location gas analysis system 200 via the network 102).

In some embodiments, one or more point location sites 106A-106N may be in fluid communication with the multipoint location gas analysis system 200. In this regard, for example, a pipe, tube, connector, any other connection means, and/or the like may provide fluid communication between the one or more point location sites 106A-106N and the multipoint location gas analysis system 200.

The multipoint location gas analysis system 200 may include circuitry, networked processors, or the like configured to perform some or all of the multipoint location gas analysis-based processes described herein and may be any suitable network server, computing device, and/or other type of processing device. In some embodiments, the multipoint location gas analysis system 200 may determine and transmit commands and instructions for rendering one or more
multipoint location gas indication status interfaces to client devices 101A-101N and/or the multipoint location gas analysis system 200 (e.g., on a user interface 210 associated with the multipoint location gas analysis system 200), using data from, for example, the multipoint location gas analysis data repository 215.

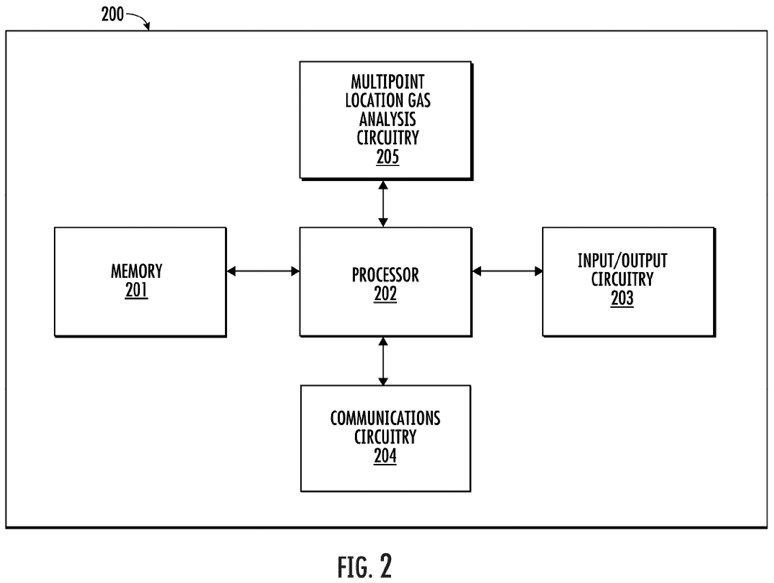

In this regard, the multipoint location gas analysis system 200 may be embodied by any of a variety of devices, for example, the multipoint location gas analysis system 200 may be embodied as a computer or a plurality of computers. For example, the multipoint location gas analysis system 200 may be configured to receive/transmit data and may include any of a variety of fixed terminals, such as a server, desktop, or kiosk, or it may comprise any of a variety of mobile terminals, such as a portable digital assistant (PDA), mobile telephone, smartphone, laptop computer, tablet computer, or in some embodiments, a peripheral device that connects to one or more fixed or mobile terminals. Example embodiments contemplated herein may have various form factors and designs but will nevertheless include at least the components illustrated in FIG. 2 and described in connection therewith. In some embodiments, multipoint location gas analysis system 200 may be located remotely from the multipoint location gas analysis data repository 215, although in other embodiments, the multipoint location gas analysis system 200 may comprise the multipoint location gas analysis data repository. The multipoint location gas analysis system 200 may, in some embodiments, comprise several servers or computing devices performing interconnected and/or distributed functions. Despite the many arrangements contemplated herein, multipoint location gas analysis system 200 is shown and described herein as a single computing device to avoid unnecessarily overcomplicating the disclosure.

The multipoint location gas analysis system 200 can communicate with one or more client devices 101A-101N via network 102. The network 102 may include any one or more wired and/or wireless communication networks including, for example, a wired or wireless local area network (LAN), personal area network (PAN), metropolitan area network (MAN), wide area network (WAN), or the like, as well as any hardware, software and/or firmware required for implementing the one or more networks (e.g., network routers, switches, hubs, etc.). For example, network 102 may include a cellular telephone, mobile broadband, long term evolution (LTE), GSM/EDGE, UMTS/HSPA, IEEE 802.11, IEEE 802.16, IEEE 802.20, Wi-Fi, dial-up, and/or WiMAX network. Furthermore, the network 102 may include a public network, such as the Internet, a private network, such as an intranet, or combinations thereof, and may utilize a variety of networking protocols now available or later developed including, but not limited to TCP/IP based networking protocols. For instance, the networking protocol may be customized to suit the needs of the multipoint location gas analysis system 200.

The multipoint location gas analysis data repository 215 may be stored by any suitable storage device configured to store some or all of the information described herein (e.g., memory 201 of the multipoint location gas analysis system 200 or a separate memory system separate from the multipoint location gas analysis system 200, such as one or more database systems, backend data servers, network databases, cloud storage devices, or the like provided by another device (e.g., online application or 3^{rd} party provider), such as a Network Attached Storage (NAS) device or devices, or as a separate database server or servers. The multipoint location gas analysis data repository 215 may comprise data received from the multipoint location gas analysis system 200 (e.g., via a memory 201 and/or processor(s) 202), and the corresponding storage device may thus store this data. The multipoint location gas analysis data repository 215 includes information accessed and stored by the multipoint location gas analysis system 200 to facilitate the operations of the multipoint location gas analysis system 200. As such, the multipoint location gas analysis data repository 215 may include, for example, without limitation, include multipoint location gas indication data, point location alert action gas data, and/or the like.

The client devices 101A-101N may be implemented as any computing device as defined above. Electronic data received by the multipoint location gas analysis system 200 from the client devices 10 1A-101N may be provided in various forms and via various methods. For example, the client devices 101A-101N may include desktop computers, laptop computers, smartphones, netbooks, tablet computers, wearables, and/or other networked device, that may be used for any suitable purpose in addition to presenting the multipoint location gas indication status interface to a user and otherwise providing access to the multipoint location gas analysis system 200. The depiction in FIG. 2 of "N" client devices is merely for illustration purposes. According to some embodiments, the client devices 101A-101N may be configured to display an interface on a display of the client device for viewing, creating, editing, and/or otherwise interacting with at least the multipoint location gas indication status interface, which may be provided by the multipoint location gas analysis system 200. According to further embodiments, the client devices 101A-101N may be configured to generate and/or display multipoint location gas indication aggregation interface components, adjusted multipoint location gas indication aggregation interface components, point location alert action gas interface components, point location service status interface components, point location maintenance fault interface components, point location instrument fault interface components, point location event interface components, and/or the like.

In embodiments where a client device 101A-101N is a mobile device, such as a smartphone or tablet, the client device 101A-101N may execute an "app" to interact with the multipoint location gas analysis system 200. Such apps are typically designed to execute on mobile devices, such as tablets or smartphones. For example, an app may be provided that executes on mobile device operating systems such as iOS^{®}, Android^{®}, or Windows^{®}. These platforms typically provide frameworks that allow apps to communicate with one another and with particular hardware and software components of mobile devices. The mobile operating systems named above each provide frameworks for interacting with, for example, wired and wireless network interfaces, user contacts, and other applications. Communication with hardware and software modules executing outside of the app is typically provided via application programming interfaces (APIs) provided by the mobile device operating system. Additionally, or alternatively, the client device 101A-101N may interact with the multipoint location gas analysis system 200 via a web browser. As yet another example, the client devices 101A-101N may include various hardware or firmware designed to interface with the multipoint location gas analysis system 200.

### Example Apparatus for Implementing Embodiments of the Present Disclosure

FIG. 2 illustrates an example multipoint location gas analysis system 200 in accordance with at least some example embodiments of the present disclosure. In accordance with some example embodiments, multipoint location gas analysis system 200may include various components, modules, circuitries, or means, some or all of which may be also or instead be included in one or more client device(s) 101A-101N. In accordance with some example embodiments, multipoint location gas analysis system 200 may be configured, using one or more of the sets of circuitry embodying memory 201, processor 202, input/output circuitry 203, communications circuitry 204, and/or multipoint location gas analysis circuitry 205 to execute and perform the operations described herein. In some embodiments, multipoint location gas analysis circuitry 205 is included in the multipoint location gas analysis system 200 and/or client device 101A, the circuitry configured to facilitate the functionality discussed herein regarding generating multipoint location gas indication aggregation interface components, adjusted multipoint location gas indication aggregation interface components, point location alert action gas interface components, point location service status interface components, point location maintenance fault interface components, point location instrument fault interface components, point location event interface components, and/or the like.

Although the use of the term "circuitry" as used herein with respect to components 201-205 are described in some cases with respect to functional limitations, it should be understood that the particular implementations necessarily include the use of particular hardware configured to perform the functions associated with the respective circuitry as described herein. It should also be understood that certain of these components 201-205 may include similar or common hardware. For example, two sets of circuitry may both leverage use of the same processor(s), network interface(s), storage medium(s), and/or the like, to perform their associated functions, such that duplicate hardware is not required for each set of circuitry. The use of the term "circuitry" with respect to components of the apparatuses described herein should therefore be understood to include particular hardware configured to perform the functions associated with the respective components or particular circuitry as described herein.

The term "circuitry" should also be understood, in some embodiments, to include software for configuring the hardware. For example, in some embodiments, "circuitry" may include processing circuitry, storage media, network interfaces, input/output devices, and the like. In some embodiments, such as in examples where circuitry is included with the multipoint location gas analysis system 200, other elements of the multipoint location gas analysis system 200 may provide or supplement the functionality of particular circuitry. For example, the processor 202 may provide processing functionality, the memory 201 may provide storage functionality, the communications circuitry 204 may provide network interface functionality, and the like.

In some embodiments, the processor 202 (and/or co-processor or any other processing circuitry assisting or otherwise associated with the processor) may be in communication with the memory 201 via a bus for passing information among components of, for example, the multipoint location gas analysis system 200. The memory 201 is non-transitory and may include, for example, one or more volatile and/or non-volatile memories, or some combination thereof. In other words, for example, the memory 201 may be an electronic storage device (e.g., a computer readable storage medium). The memory 201 may be configured to store information, data, content, applications, instructions, or the like, for enabling an apparatus, e.g., the multipoint location gas analysis system 200 to carry out various functions in accordance with example embodiments of the present disclosure.

Although illustrated in FIG. 2 as a single memory, memory 201 may comprise a plurality of memory components. The plurality of memory components may be embodied on a single computing device or distributed across a plurality of computing devices. In various embodiments, memory 201 may comprise, for example, a hard disk, random access memory, cache memory, flash memory, a compact disc read only memory (CD-ROM), digital versatile disc read only memory (DVD-ROM), an optical disc, circuitry configured to store information, or some combination thereof. Memory 201 may be configured to store information, data, applications, instructions, or the like for enabling the multipoint location gas analysis system 200 to carry out various functions in accordance with example embodiments discussed herein. For example, in at least some embodiments, memory 201 is configured to buffer data for processing by processor 202. Additionally, or alternatively, in at least some embodiments, memory 201 is configured to store program instructions for execution by processor 202. Memory 201 may store information in the form of static and/or dynamic information. This stored information may be stored and/or used by the multipoint location gas analysis system 200 during the course of performing its functionalities.

Processor 202 may be embodied in a number of different ways and may, for example, include one or more processing devices configured to perform independently. Additionally, or alternatively, processor 202 may include one or more processors configured in tandem via a bus to enable independent execution of instructions, pipelining, and/or multithreading. Processor 202 may, for example, be embodied as various means including one or more microprocessors with accompanying digital signal processor(s), one or more processor(s) without an accompanying digital signal processor, one or more coprocessors, one or more multi-core processors, one or more controllers, processing circuitry, one or more computers, various other processing elements including integrated circuits such as, for example, an ASIC (application specific integrated circuit) or FPGA (field programmable gate array), or some combination thereof. The use of the term "processing circuitry" may be understood to include a single core processor, a multi-core processor, multiple processors internal to the apparatus, and/or remote or "cloud" processors. Accordingly, although illustrated in FIG. 2 as a single processor, in some embodiments, processor 202 comprises a plurality of processors. The plurality of processors may be embodied on a single computing device or may be distributed across a plurality of such devices collectively configured to function as the multipoint location gas analysis system 200. The plurality of processors may be in operative communication with each other and may be collectively configured to perform one or more functionalities of the multipoint location gas analysis system 200 as described herein.

In an example embodiment, processor 202 is configured to execute instructions stored in the memory 201 or otherwise accessible to processor 202. Alternatively, or additionally, the processor 202 may be configured to execute hard-coded functionality. As such, whether configured by hardware or software methods, or by a combination thereof, the processor 202 may represent an entity (e.g., physically embodied in circuitry) capable of performing operations according to an embodiment of the present disclosure while configured accordingly. Alternatively, as another example, when the processor 202 is embodied as an executor of software instructions, the instructions may specifically configure processor 202 to perform one or more algorithms and/or operations described herein when the instructions are executed. For example, these instructions, when executed by processor 202, may cause the multipoint location gas analysis system 200 to perform one or more of the functionalities of the multipoint location gas analysis system 200 as described herein.

In some embodiments, input/output circuitry 203 may, in turn, be in communication with processor 202 to provide an audible, visual, mechanical, or other output and/or, in some embodiments, to receive an indication of an input. In that sense, input/output circuitry 203 may include means for performing analog-to-digital and/or digital-to-analog data conversions. Input/output circuitry 203 may include support, for example, for a display, touchscreen, keyboard, button, click wheel, mouse, joystick, an image capturing device (e.g., a camera), motion sensor (e.g., accelerometer and/or gyroscope), microphone, audio recorder, speaker, biometric scanner, and/or other input/output mechanisms. Input/output circuitry 203 may comprise a user interface (e.g., a multipoint location gas indication status interface) and may comprise a web user interface, a mobile application, a kiosk, or the like. The processor 202 and/or user interface circuitry comprising the processor 202 may be configured to control one or more functions of a display or one or more user interface elements through computer program instructions (e.g., software and/or firmware) stored on a memory accessible to the processor 202 (e.g., memory 201, and/or the like). In some embodiments, aspects of input/output circuitry 203 in the multipoint location gas analysis system 200 may be reduced when implemented also or instead as an end-user machine or other type of device designed for complex user interactions (i.e., client device 101). In some embodiments (like other components discussed herein), input/output circuitry 203 may even be eliminated from the multipoint location gas analysis system 200. Alternatively, at least some aspects of input/output circuitry 203 may be embodied on an apparatus used by a user that is in communication with the multipoint location gas analysis system 200. Input/output circuitry 203 may be in communication with memory 201, communications circuitry 204, and/or any other component(s), such as via a bus. Although more than one input/output circuitry and/or other component can be included in the multipoint location gas analysis system 200, only one is shown in FIG. 2 to avoid overcomplicating the disclosure (e.g., like the other components discussed herein).

Communications circuitry 204 may be any means such as a device or circuitry embodied in either hardware or a combination of hardware and software that is configured to receive and/or transmit data from/to a network and/or any other device, circuitry, or module in communication with the multipoint location gas analysis system 200. In this regard, the communications circuitry 204 may include, for example, a network interface for enabling communications with a wired or wireless communication network. Communications circuitry 204 may be configured to receive and/or transmit any data that may be stored by memory 201 using any protocol that may be used for communications between computing devices. For example, the communications circuitry 204 may include one or more network interface cards, antennae, transmitters, receivers, buses, switches, routers, modems, and supporting hardware and/or software, and/or firmware/software, or any other device suitable for enabling communications via a network. Additionally, or alternatively, the communication interface may include the circuitry for interacting with the antenna(s) to cause transmission of signals via the antenna(s) or to handle receipt of signals received via the antenna(s). These signals may be transmitted by the multipoint location gas analysis system 200 using any of a number of wireless personal area network (PAN) technologies, such as Bluetooth^{®} v1.0 through v3.0, Bluetooth Low Energy (BLE), infrared wireless (e.g., IrDA), ultra-wideband (UWB), induction wireless transmission, or the like. In addition, it should be understood that these signals may be transmitted using Wi-Fi, Near Field Communications (NFC), Worldwide Interoperability for Microwave Access (WiMAX) or other proximity-based communications protocols. Communications circuitry 204 may additionally or alternatively be in communication with the memory 201, input/output circuitry 203 and/or any other component of the multipoint location gas analysis system 200, such as via a bus.

In some embodiments, multipoint location gas analysis circuitry 205 may also or instead be included and configured to perform the functionality discussed herein related to providing multipoint location gas indication status interfaces, multipoint location gas indication aggregation interface components, adjusted multipoint location gas indication aggregation interface components, point location alert action gas interface components, point location service status interface components, point location maintenance fault interface components, point location instrument fault interface components, point location event interface components, and/or the like. Multipoint location gas analysis circuitry 205 includes hardware components and/or software configured to support interface component functionality, features, and/or services of the multipoint location gas analysis system 200. The multipoint location gas analysis circuitry 205 may utilize processing circuitry, such as the processor 202, to perform its corresponding operations, and may utilize memory 201 to store collected information. The multipoint location gas analysis circuitry 205 may send and/or receive data from the multipoint location gas analysis data repository 215. In some implementations, the sent and/or received data may include multipoint location gas indication data, point location alert action gas data, and/or associated data that is associated with, for example, generating multipoint location gas indication status interfaces, multipoint location gas indication aggregation interface components, adjusted multipoint location gas indication aggregation interface components, point location alert action gas interface components, point location service status interface components, point location maintenance fault interface components, point location instrument fault interface components, point location event interface components, and/or the like.

It should also be appreciated that, in some embodiments, the multipoint location gas analysis circuitry 205 may include a separate processor, specially configured field programmable gate array (FPGA), or application specific interface circuit (ASIC) to perform its corresponding functions. For example, in some embodiments, some or all of the functionality of multipoint location gas analysis circuitry 205 may be performed by processor 202. In this regard, some or all of the example processes and algorithms discussed herein can be performed by at least one processor 202 and/or multipoint location gas analysis circuitry 205. For example, non-transitory computer readable storage media can be configured to store firmware, one or more application programs, and/or other software, which include instructions and other computer-readable program code portions that can be executed to control processors of the components of the multipoint location gas analysis system 200 to implement various operations, including the examples shown herein. As such, a series of computer-readable program code portions may be embodied in one or more computer program products and can be used, with a device, multipoint location gas analysis system 200, database, and/or other programmable apparatus, to produce the machine-implemented processes discussed herein. It is also noted that all or some of the information discussed herein can be based on data that is received, generated and/or maintained by one or more components of the multipoint location gas analysis system 200. In some embodiments, one or more external systems (such as a remote cloud computing and/or data storage system) may also be leveraged to provide at least some of the functionality discussed herein.

As described above and as will be appreciated based on this disclosure, embodiments of the present disclosure may be configured as systems, methods, apparatuses, computing devices, personal computers, servers, mobile devices, backend network devices, and/or the like. Accordingly, embodiments may comprise various means including entirely of hardware or any combination of software and hardware. Furthermore, embodiments may take the form of a computer program product on at least one non-transitory computer-readable storage medium having computer-readable program instructions embodied in the computer-readable storage medium (e.g., computer software stored on a hardware device). Any suitable computer-readable storage medium may be utilized including non-transitory hard disks, CD-ROMs, flash memory, optical storage devices, or magnetic storage devices.

As will be appreciated, any such computer program instructions and/or other type of code may be loaded onto a computer, processor, or other programmable apparatus's circuitry to produce a machine, such that the computer, processor, or other programmable circuitry that execute the code on the machine creates the means for implementing various functions, including those described herein in connection with the components of the multipoint location gas analysis system 200.

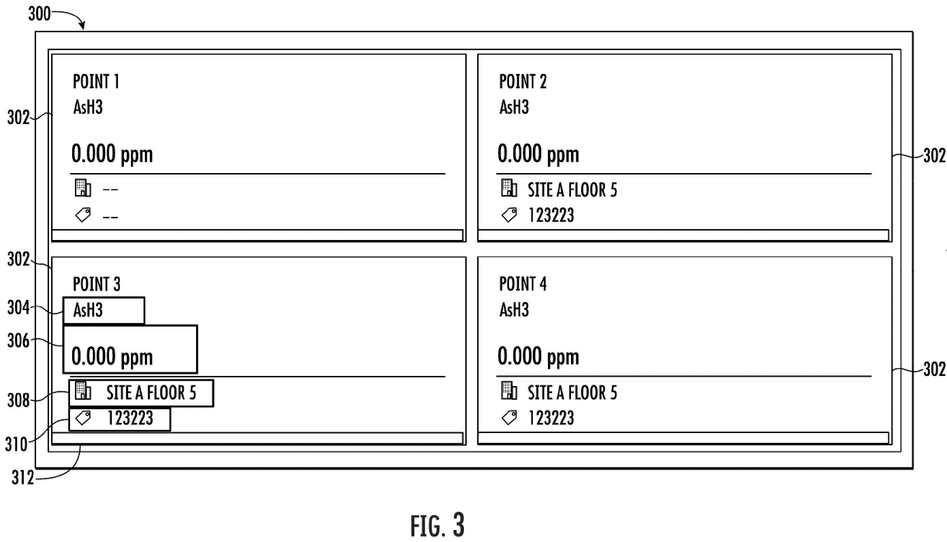

In various embodiments of the present disclosure, a system (e.g., the multipoint location gas analysis system 200) may be configured to generate, output, manage, and/or cause rendering of a multipoint location gas indication aggregation interface component of a multipoint location gas analysis system 200. For example, a system (e.g., the multipoint location gas analysis system 200) may be configured to generate, output, manage, and/or cause rendering of a multipoint location gas indication aggregation interface component on a multipoint location gas indication status interface of a multipoint location gas analysis system 200. FIG. 3 illustrates an example multipoint location gas indication aggregation interface component 300 structured in accordance with various embodiments of the present disclosure. In some embodiments, the system (e.g., multipoint location gas analysis system 200) causes rendering of the multipoint location gas indication aggregation interface component 300 to a visual display of a computing device (e.g., a client device 101A-101N and/or the multipoint location has analysis system 200).

In some embodiments, the multipoint location gas indication aggregation interface component 300 may be configured to display one or more point location gas indication interface components. For example, the multipoint location gas indication aggregation interface component 300 may be configured to display a plurality of point location gas indication interface components 302. Although in the non-limiting example depicted in FIG. 3, the multipoint location gas indication aggregation interface component 300 includes four point location gas indication interface components, it would be understood by one skilled in the field to which this disclosure pertains that the multipoint location gas indication aggregation interface component 300 may be configured to display any number of point location gas indication interface components.

In some embodiments, each of the plurality of point location gas indication interface components 302 may be configured to display a point location gas type interface component. For example, each of the plurality of point location gas indication interface components 302 may be configured to display a point location gas type interface component 304. In some embodiments, the point location gas type interface component 304 may be configured to display text, color, graphics, and/or the like representative of a point location gas type identifier (e.g., text comprising AsH3).

In some embodiments, each of the plurality of point location gas indication interface components 302 may be configured to display a point location gas concentration interface component. For example, each of the plurality of point location gas indication interface components 302 may be configured to display a point location gas concentration interface component 306. In some embodiments, the point location gas concentration interface component 306 may be configured to display text, color, graphics, and/or the like representative of a point location gas concentration indicator (e.g., text comprising 0.000 ppm).

In some embodiments, each of the plurality of point location gas indication interface components 302 may be configured to display a point location site interface component. For example, each of the plurality of point location gas indication interface components 302 may be configured to display a point location site interface component 308. In some embodiments, the point location site interface component 308 may be configured to display text, color, graphics, and/or the like representative of a point location site identifier (e.g., text comprising Site A Floor Five).

In some embodiments, each of the plurality of point location gas indication interface components 302 may be configured to display a point location unique identification interface component. For example, each of the plurality of point location gas indication interface components 302 may be configured to display a point location unique identification interface component 310. In some embodiments, the point location unique identification interface component 310 may be configured to display text, color, graphics, and/or the like representative of a point location unique identification identifier (e.g., text comprising 123223).

In some embodiments, each of the plurality of point location gas indication interface components 302 may be configured to display a point location status interface component. For example, each of the plurality of point location gas indication interface components 302 may be configured to display a point location status interface component 312. In some embodiments, the point location status interface component 312 may be configured to display text, color, graphics, and/or the like representative of a point location status indicator (e.g., text stating that there is an acceptable gas concentration level).

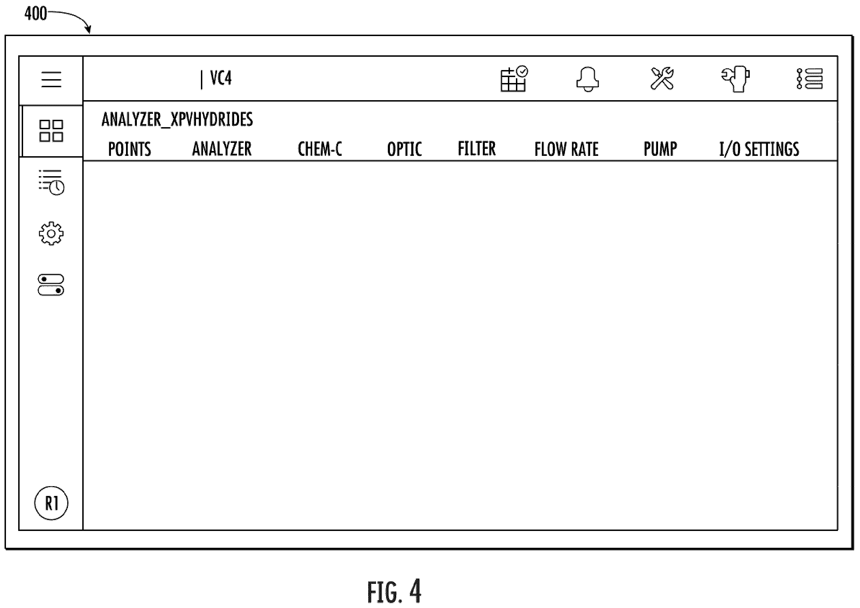

In various embodiments of the present disclosure, a system (e.g., the multipoint location gas analysis system 200) may be configured to generate, output, manage, and/or cause rendering of a multipoint location gas indication status interface of a multipoint location gas analysis system 200. For example, a system (e.g., the multipoint location gas analysis system 200) may be configured to generate, output, manage, and/or cause rendering of a multipoint location gas indication aggregation interface component on a user interface 210 of a multipoint location gas analysis system 200. FIG. 4 illustrates an example multipoint location gas indication status interface 400 structured in accordance with various embodiments of the present disclosure. In some embodiments, the system (e.g., multipoint location gas analysis system 200) causes rendering of the multipoint location gas indication status interface 400 to a visual display of a computing device (e.g., a client device 101A-101N and/or the multipoint location has analysis system 200).

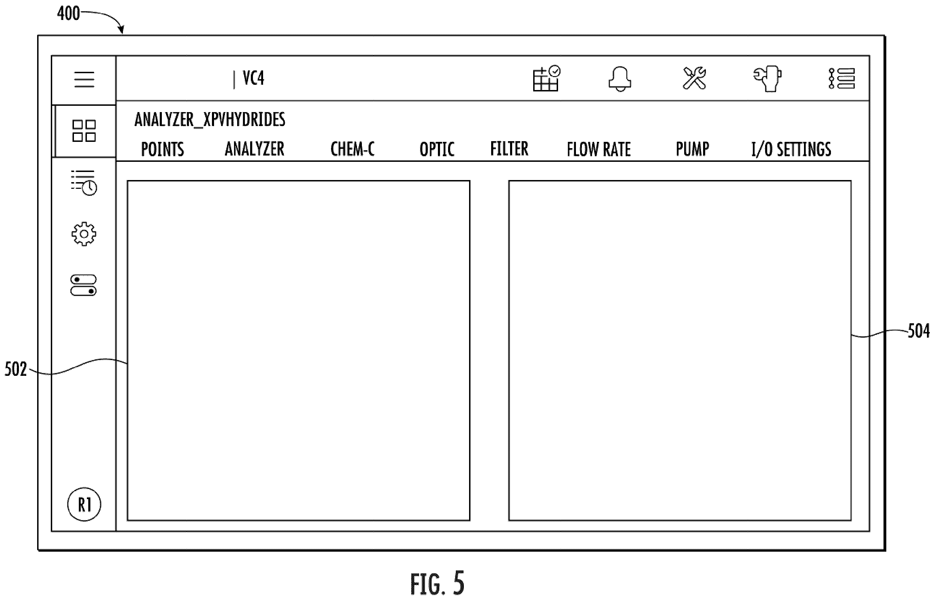

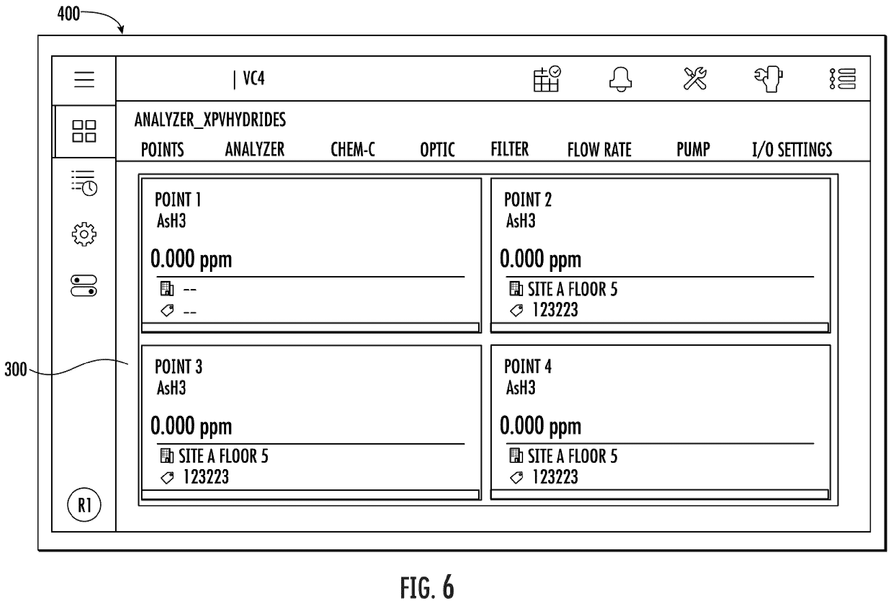

In some embodiments, the multipoint location gas indication status interface 400 may comprise a first multipoint location gas indication status portion and/or a second multipoint location gas indication status portion. FIG. 5 illustrates an example first multipoint location gas indication status portion 502 and an example second multipoint location gas indication status portion 504. In some embodiments, such as illustrated in FIG. 6, the multipoint location gas indication aggregation interface component 300 may be configured to be rendered to the first multipoint location gas indication status portion 502 of the multipoint location gas indication status interface 400 and the second multipoint location gas indication status portion 504 of the multipoint location gas indication status interface 400. Said differently, the multipoint location gas indication aggregation interface component 300 may be configured such that at least a portion of the multipoint location gas indication aggregation interface component 300 is rendered on the first multipoint location gas indication status portion 502 and at least another portion of the multipoint location gas indication aggregation interface component 300 is rendered on the second multipoint location gas indication status portion 504.

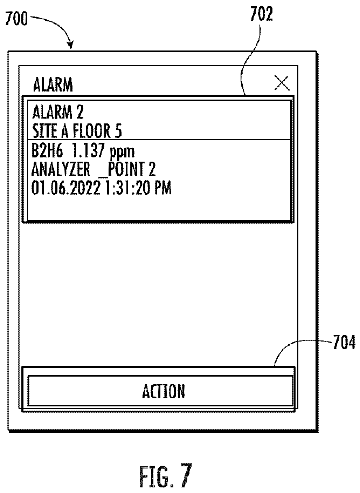

In various embodiments of the present disclosure, a system (e.g., the multipoint location gas analysis system 200) may be configured to generate, output, manage, and/or cause rendering of a point location alert gas interface component of a multipoint location gas analysis system 200. For example, a system (e.g., the multipoint location gas analysis system 200) may be configured to generate, output, manage, and/or cause rendering of a point location alert gas interface component on a multipoint location gas indication status interface of a multipoint location gas analysis system 200. FIG. 7 illustrates an example point location alert gas interface component 700 structured in accordance with various embodiments of the present disclosure. In some embodiments, the system (e.g., multipoint location gas analysis system 200) causes rendering of the point location alert gas interface component 700 to a visual display of a computing device (e.g., a client device 101A-101N and/or the multipoint location has analysis system 200).

In some embodiments, the point location alert gas interface component 700 may be configured to display a point location alert gas subsidiary interface component. For example, the point location alert gas interface component 700 may be configured to display a point location alert gas subsidiary interface component 702. Although in the non-limiting example depicted in FIG. 7, the point location alert gas interface component 700 includes one point location alert gas subsidiary interface component, it would be understood by one skilled in the field to which this disclosure pertains that the point location alert gas interface component 700 may be configured to display any number of point location alert gas subsidiary interface components.

In some embodiments, the point location alert gas subsidiary interface component 702 may be configured to display text, colors, symbols, and/or the like representative of one or more of a point location site identifier, a point location unique identification identifier, a point location gas concentration indicator, a point location gas type identifier, and/or a point location status indicator associated with a point location site (e.g., one of point location sites 106A-106N) associated with a gas anomaly event. For example, for a point location site associated with a point location gas concentration greater than a point location gas concentration threshold, the point location alert gas subsidiary interface component 702 may be configured to display text, colors, symbols, and/or the like representative of a point location gas concentration identifier associated with the point location site.

In some embodiments, the point location alert gas interface component 700 may be configured to display a point location alert action gas interface component. For example, the point location alert gas interface component 700 may be configured to display a point location alert action gas interface component 704. Although in the non-limiting example depicted in FIG. 7, the point location alert gas interface component 700 includes one point location alert action gas interface component, it would be understood by one skilled in the field to which this disclosure pertains that the point location alert gas interface component 700 may be configured to display any number of point location alert action gas interface components.

In some embodiments, the point location alert action gas interface component 704 may be configured to be selected by a user such that a user can select the point location alert action gas interface component to acknowledge that a gas anomaly event has occurred. In some embodiments, a point location alert action gas interface component may be configured to be selected by a user such that a user can trigger one or more actions in response to a gas anomaly event by selecting the point location alert action gas interface component. For example, the one or more actions that can be triggered in response to a gas anomaly event by a user selecting a point location alert action gas interface component may include triggering an alarm to inform individuals associated with the point location site of the gas anomaly event. As another example, the one or more actions that can be triggered in response to a gas anomaly event by a user selecting a point location alert action gas interface component may include causing an evacuation of the point location site. As another example, the one or more actions that can be triggered in response to a gas anomaly event by a user selecting a point location alert action gas interface component may include causing machinery and/or processes associated with the point location site to shut down.

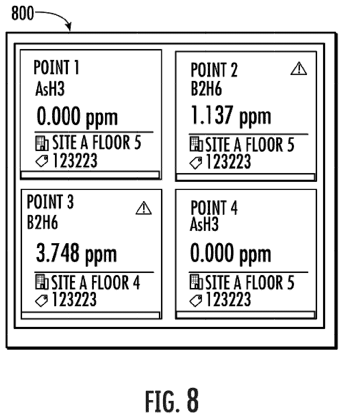

In various embodiments of the present disclosure, a system (e.g., the multipoint location gas analysis system 200) may be configured to generate, output, manage, and/or cause rendering of an adjusted multipoint location gas indication aggregation interface component of a multipoint location gas analysis system 200. For example, a system (e.g., the multipoint location gas analysis system 200) may be configured to generate, output, manage, and/or cause rendering of an adjusted multipoint location gas indication aggregation interface component on a multipoint location gas indication status interface of a multipoint location gas analysis system 200. FIG. 8 illustrates an example adjusted multipoint location gas indication aggregation interface component 800 structured in accordance with various embodiments of the present disclosure. In some embodiments, the system (e.g., multipoint location gas analysis system 200) causes rendering of the adjusted multipoint location gas indication aggregation interface component 800 to a visual display of a computing device (e.g., a client device 101A-101N and/or the multipoint location has analysis system 200).

In some embodiments, the adjusted multipoint location gas indication aggregation interface component 800 may be configured to display one or more point location gas indication interface components. For example, the adjusted multipoint location gas indication aggregation interface component 800 may be configured to display a plurality of point location gas indication interface components 302. Although in the non-limiting example depicted in FIG. 8, the adjusted multipoint location gas indication aggregation interface component 800 includes four point location gas indication interface components, it would be understood by one skilled in the field to which this disclosure pertains that the adjusted multipoint location gas indication aggregation interface component 800 may be configured to display any number of point location gas indication interface components.

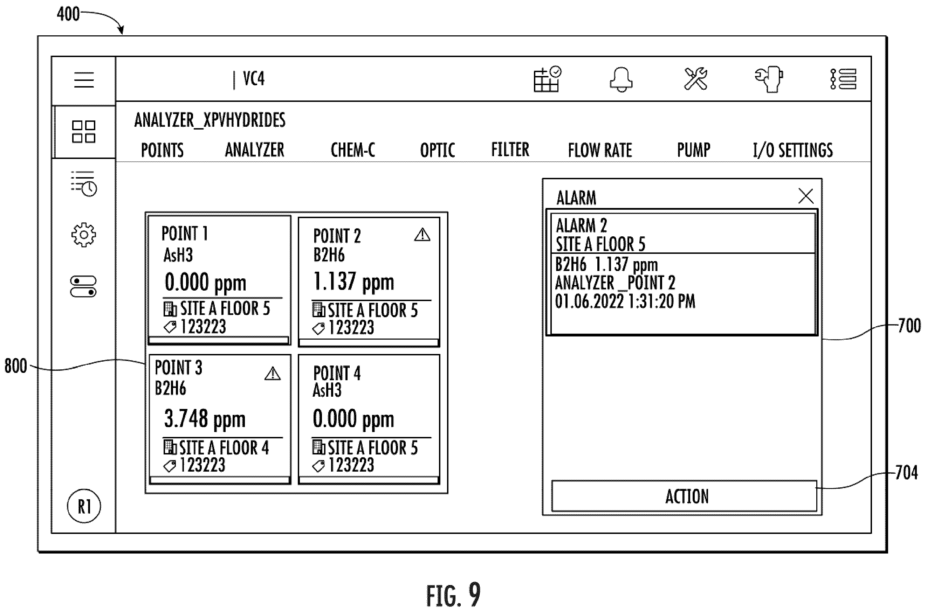

In some embodiments, such as illustrated in FIG. 9, the adjusted multipoint location gas indication aggregation interface component 800 may be configured to be rendered to the first multipoint location gas indication status portion 502 of the multipoint location gas indication status interface 400. In some embodiments, such as illustrated in FIG. 9, the point location alert gas interface component 700 may be configured to be rendered to the second multipoint location gas indication status portion 504 of the multipoint location gas indication status interface 400. In this regard, the adjusted multipoint location gas indication aggregation interface component 800 may be rendered proximate the point location alert gas interface component 700 on the multipoint location gas indication status interface 400.

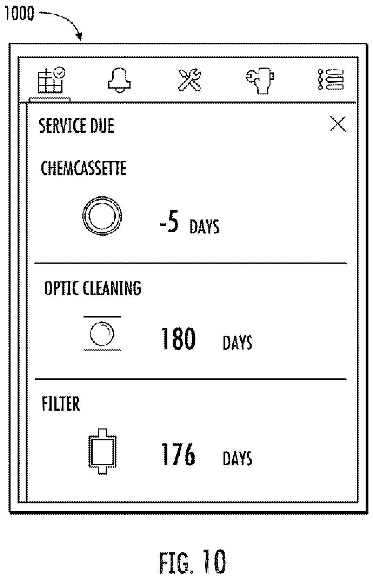

In various embodiments of the present disclosure, a system (e.g., the multipoint location gas analysis system 200) may be configured to generate, output, manage, and/or cause rendering of a point location service status interface component of a multipoint location gas analysis system 200. For example, a system (e.g., the multipoint location gas analysis system 200) may be configured to generate, output, manage, and/or cause rendering of a point location service status interface component on a multipoint location gas indication status interface of a multipoint location gas analysis system 200. FIG. 10 illustrates an example point location service status interface component 1000 structured in accordance with various embodiments of the present disclosure. In some embodiments, the system (e.g., multipoint location gas analysis system 200) causes rendering of the point location service status interface component 1000 to a visual display of a computing device (e.g., a client device 101A-101N and/or the multipoint location has analysis system 200).

In some embodiments, the point location service status interface component 1000 may be configured to display text, colors, symbols, and/or the like representative of one or more service statuses associated with the multipoint location gas analysis system 200. In some embodiments, for example, the point location service status interface component 1000 may be configured to display text, colors, symbols, and/or the like representative of one or more service statuses associated with the multipoint location gas analysis system 200. In some embodiments, for example, the point location service status interface component 1000 may be configured to display text, colors, symbols, and/or the like representative of an age of a Chemcassette^{®} paper associated with the multipoint location gas analysis system 200 (e.g., 5 days). As another example, the point location service status interface component 1000 may be configured to display text, colors, symbols, and/or the like representative of an optic cleaning timeline associated with the multipoint location gas analysis system 200 (e.g., 180 days). As another example, the point location service status interface component 1000 may be configured to display text, colors, symbols, and/or the like representative of a filter age associated with the multipoint location gas analysis system 200 (e.g., 176 days).

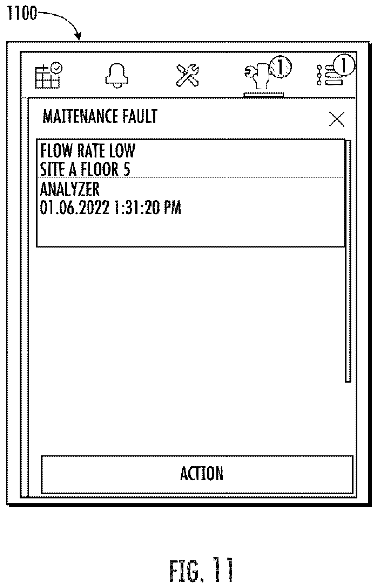

In various embodiments of the present disclosure, a system (e.g., the multipoint location gas analysis system 200) may be configured to generate, output, manage, and/or cause rendering of a point location maintenance fault interface component of a multipoint location gas analysis system 200. For example, a system (e.g., the multipoint location gas analysis system 200) may be configured to generate, output, manage, and/or cause rendering of a point location maintenance fault interface component on a multipoint location gas indication status interface of a multipoint location gas analysis system 200. FIG. 11 illustrates an example point location maintenance fault interface component 1100 structured in accordance with various embodiments of the present disclosure. In some embodiments, the system (e.g., multipoint location gas analysis system 200) causes rendering of the point location maintenance fault interface component 1100 to a visual display of a computing device (e.g., a client device 101A-101N and/or the multipoint location has analysis system 200).

In some embodiments, the point location maintenance fault interface component 1100 may be configured to display text, colors, symbols, and/or the like representative of one or more maintenance faults associated with the multipoint location gas analysis system 200. For example, the point location maintenance fault interface component 1100 may be configured to display text, colors, symbols, and/or the like representative of a low flow rate maintenance fault.

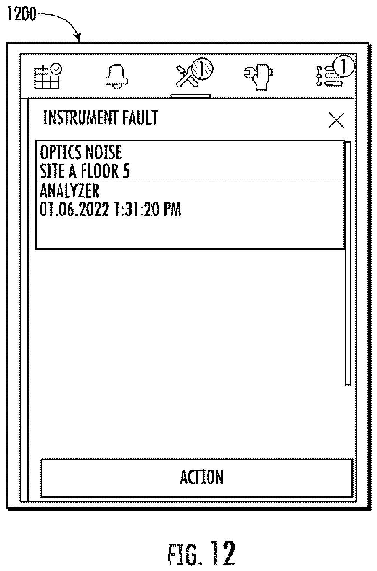

In various embodiments of the present disclosure, a system (e.g., the multipoint location gas analysis system 200) may be configured to generate, output, manage, and/or cause rendering of a point location instrument fault interface component of a multipoint location gas analysis system 200. For example, a system (e.g., the multipoint location gas analysis system 200) may be configured to generate, output, manage, and/or cause rendering of a point location instrument fault interface component on a multipoint location gas indication status interface of a multipoint location gas analysis system 200. FIG. 12 illustrates an example point location instrument fault interface component 1200 structured in accordance with various embodiments of the present disclosure. In some embodiments, the system (e.g., multipoint location gas analysis system 200) causes rendering of the point location instrument fault interface component 1200 to a visual display of a computing device (e.g., a client device 101A-101N and/or the multipoint location has analysis system 200).

In some embodiments, the point location instrument fault interface component 1200 may be configured to display text, colors, symbols, and/or the like representative of one or more instrument faults associated with the multipoint location gas analysis system 200. For example, the point location instrument fault interface component 1200 may be configured to display text, colors, symbols, and/or the like representative of an optic noise instrument fault (e.g., noise associated with optics configured to perform an optical analysis technique).

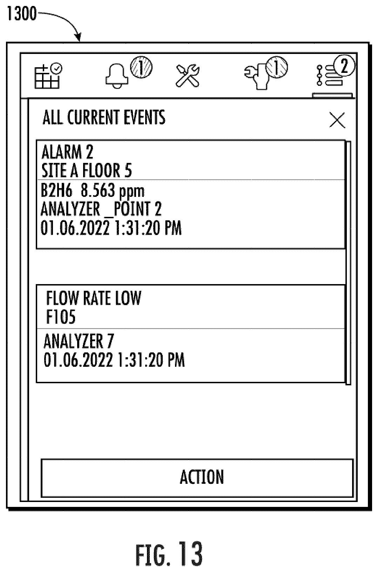

In various embodiments of the present disclosure, a system (e.g., the multipoint location gas analysis system 200) may be configured to generate, output, manage, and/or cause rendering of a point location event interface component of a multipoint location gas analysis system 200. For example, a system (e.g., the multipoint location gas analysis system 200) may be configured to generate, output, manage, and/or cause rendering of a point location event interface component on a multipoint location gas indication status interface of a multipoint location gas analysis system 200. FIG. 13 illustrates an example point location event interface component 1300 structured in accordance with various embodiments of the present disclosure. In some embodiments, the system (e.g., multipoint location gas analysis system 200) causes rendering of the point location event interface component 1300 to a visual display of a computing device (e.g., a client device 101A-101N and/or the multipoint location has analysis system 200).

In some embodiments, the point location event interface component 1300 may be configured to display text, colors, symbols, and/or the like representative of one or more events associated with the multipoint location gas analysis system 200. For example, the point location event interface component 1300 may be configured to display text, colors, symbols, and/or the like representative of an event that comprises a gas anomaly event associated with the multipoint location gas analysis system 200. As another example, the point location event interface component 1300 may be configured to display text, colors, symbols, and/or the like representative of a maintenance fault associated with the multipoint location gas analysis system 200 (e.g., a low flow rate maintenance fault).

In some embodiments, the system (e.g., multipoint location gas analysis system 200) may be configured to generate, output, manage, and/or cause rendering of at least a portion of the multipoint location gas indication aggregation interface component 300, the multipoint location gas indication status interface 400, point location alert gas interface component 700, the adjusted multipoint location gas indication aggregation interface component 800, the point location service status interface component 1000, the point location maintenance fault interface component 1100, the point location instrument fault interface component 1200, and/or the point location event interface component 1300 in a deuteranopia type configuration.

In some embodiments, the system (e.g., multipoint location gas analysis system 200) may be configured to generate, output, manage, and/or cause rendering of at least a portion of the multipoint location gas indication aggregation interface component 300, the multipoint location gas indication status interface 400, point location alert gas interface component 700, the adjusted multipoint location gas indication aggregation interface component 800, the point location service status interface component 1000, the point location maintenance fault interface component 1100, the point location instrument fault interface component 1200, and/or the point location event interface component 1300 in a protanopia type configuration.

In some embodiments, the system (e.g., multipoint location gas analysis system 200) may be configured to generate, output, manage, and/or cause rendering of at least a portion of the multipoint location gas indication aggregation interface component 300, the multipoint location gas indication status interface 400, point location alert gas interface component 700, the adjusted multipoint location gas indication aggregation interface component 800, the point location service status interface component 1000, the point location maintenance fault interface component 1100, the point location instrument fault interface component 1200, and/or the point location event interface component 1300 in a tritanopia type configuration.

In some embodiments, the system (e.g., multipoint location gas analysis system 200) may be configured to generate, output, manage, and/or cause rendering of at least a portion of the multipoint location gas indication aggregation interface component 300, the multipoint location gas indication status interface 400, point location alert gas interface component 700, the adjusted multipoint location gas indication aggregation interface component 800, the point location service status interface component 1000, the point location maintenance fault interface component 1100, the point location instrument fault interface component 1200, and/or the point location event interface component 1300 in a monochromacy type configuration.

### Example Operations Performed

Having described example systems and apparatuses and exemplary circuitry in accordance with various embodiments of the present disclosure, example processes of the disclosure will now be discussed. It will be appreciated that each of the flowcharts depicts an example computer-implemented process that is performable by one or more of the apparatuses, systems, devices, and/or computer program products described herein, for example utilizing one or more of the specially configured components thereof. It will further be appreciated that the example apparatuses, systems, devices, and/or computer program products may proceed to output and/or provide multipoint location gas indication status interfaces, multipoint location gas indication aggregation interface components, adjusted multipoint location gas indication aggregation interface components, point location alert gas interface components, point location service status interface components, point location maintenance fault interface components, point location instrument fault interface components, point location event interface components, and/or the like in a multipoint location gas analysis system in a number of ways.

The blocks indicate operations of each process. Such operations may be performed in any of a number of ways, including, without limitation, in the order and manner as depicted and described herein. In some embodiments, one or more blocks of any of the processes described herein occur in-between one or more blocks of another process, before one or more blocks of another process, in parallel with one or more blocks of another process, and/or as a sub-process of a second process. Additionally, or alternatively, any of the processes in various embodiments include some or all operational steps described and/or depicted, including one or more optional blocks in some embodiments. With regard to the flowcharts illustrated herein, one or more of the depicted block(s) in some embodiments is/are optional in some, or all, embodiments of the disclosure. Optional blocks are depicted with broken (or "dashed") lines. Similarly, it should be appreciated that one or more of the operations of each flowchart may be combinable, replaceable, and/or otherwise altered as described herein.

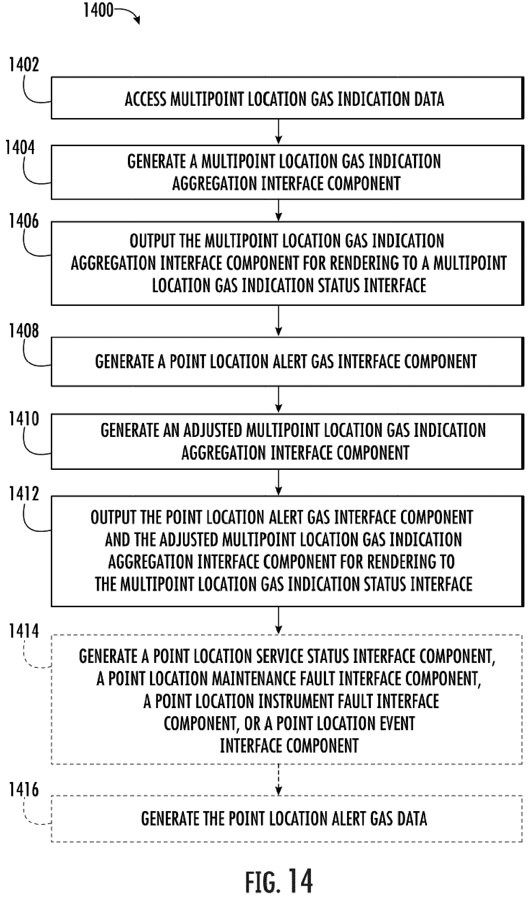

FIG. 14 is a flowchart broadly illustrating a series of operations or process blocks that are executed or performed to output a point location alert gas interface component and an adjusted multipoint location gas indication aggregation interface component for rendering to a multipoint location gas indication status interface of the computing device associated with the multipoint location gas indication aggregation interface component in a multipoint location gas analysis system in accordance with some example embodiments of the present disclosure. In some embodiments, the method 1400 is embodied by computer program code stored on a non-transitory computer-readable storage medium of a computer program product configured for execution to perform the process as depicted and described. In this regard, in some such embodiments, the multipoint location gas analysis system 200 is specially configured by computer-coded instructions (e.g., computer program instructions) stored thereon, for example in the memory 201 and/or another component depicted and/or described herein and/or otherwise accessible to the multipoint location gas analysis system 200, for performing the operations as depicted and described. Alternatively, or additionally, in some embodiments, the method 1400 is performed by one or more specially configured computing devices, such as the multipoint location gas analysis system 200 alone or in communication with one or more other component(s), device(s), system(s), and/or the like. For example, in some embodiments, the multipoint location gas analysis system 200 is in communication with one or more external apparatus(es), system(s), device(s), and/or the like, to perform one or more of the operations as depicted and described. For purposes of simplifying the description, the method 1400 is described as performed by and from the perspective of the multipoint location gas analysis system 200. In this regard, performance of the operations may invoke one or more of memory 201, processor 202, input/output circuitry 203, communications circuitry 204, and/or multipoint location gas analysis circuitry 205.

In an embodiment illustrated in FIG. 14, the flowchart illustrated method 1400 which begins at block 1402. At block 1402, the multipoint location gas analysis system 200 includes means, such as the memory 201, processor 202, input/output circuitry 203, communications circuitry 204, and/or multipoint location gas analysis circuitry 205, or a combination thereof, to access multipoint location gas indication data. In this regard, for example, the multipoint location gas analysis system 200 may access one or more items of data indicative of a plurality of point location site identifiers, a plurality of point location unique identification identifiers, a plurality of point location gas concentration indicators, a plurality of point location gas type identifiers, and/or a plurality of point location status indicators. In some embodiments, the multipoint location gas analysis system 200 may access multipoint location gas indication data from the multipoint location gas analysis data repository 215.

At block 1404, the multipoint location gas analysis system 200 includes means, such as the memory 201, processor 202, input/output circuitry 203, communications circuitry 204, and/or multipoint location gas analysis circuitry 205, or a combination thereof, to generate a multipoint location gas indication aggregation interface component. In some embodiments, an adjusted multipoint location gas indication aggregation interface component may be configured to display a plurality of point location gas indication interface components. For example, an adjusted multipoint location gas indication aggregation interface component may be configured to display four point location gas indication interface components. In some embodiments, a multipoint location gas indication aggregation interface component may be based at least in part on multipoint location gas indication data and/or point location alert gas data.

At block 1406, the multipoint location gas analysis system 200 includes means, such as the memory 201, processor 202, input/output circuitry 203, communications circuitry 204, and/or multipoint location gas analysis circuitry 205, or a combination thereof, to output the multipoint location gas indication aggregation interface component for rendering to a multipoint location gas indication status interface. In some embodiments, the multipoint location gas indication status interface may be configured to enable one or more users to view and engage with one or more multipoint location gas analysis system, views, and/or interface components. In some embodiments, for example, a multipoint location gas indication status interface is configured to enable one or more users to view and engage with one or more of a multipoint location gas indication aggregation interface component, an adjusted multipoint location gas indication aggregation interface component, a point location alert gas interface component, a point location service status interface component, a point location maintenance fault interface component, a point location instrument fault interface component, and/or a point location event interface component.

At block 1408, the multipoint location gas analysis system 200 includes means, such as the memory 201, processor 202, input/output circuitry 203, communications circuitry 204, and/or multipoint location gas analysis circuitry 205, or a combination thereof, to generate a point location alert gas interface component. In some embodiments, a point location alert gas interface component may be configured to display one or more point location alert gas subsidiary interface components and/or one or more point location alert action gas interface components. In some embodiments, a point location alert gas interface component may be based at least in part on multipoint location gas indication data and/or point location alert gas data.

At block 1410, the multipoint location gas analysis system 200 includes means, such as the memory 201, processor 202, input/output circuitry 203, communications circuitry 204, and/or multipoint location gas analysis circuitry 205, or a combination thereof, to generate an adjusted multipoint location gas indication aggregation interface component. In some embodiments, an adjusted multipoint location gas indication aggregation interface component may be configured to display a plurality of point location gas indication interface components. For example, an adjusted multipoint location gas indication aggregation interface component may be configured to display four point location gas indication interface components. In some embodiments, a multipoint location gas indication aggregation interface component may be based at least in part on multipoint location gas indication data and/or point location alert gas data.

At block 1412, the multipoint location gas analysis system 200 includes means, such as the memory 201, processor 202, input/output circuitry 203, communications circuitry 204, and/or multipoint location gas analysis circuitry 205, or a combination thereof, to output the point location alert gas interface component and the adjusted multipoint location gas indication aggregation interface component for rendering to the multipoint location gas indication status interface. In some embodiments, the multipoint location gas indication status interface may be configured to enable one or more users to view and engage with one or more multipoint location gas analysis system, views, and/or interface components. In some embodiments, for example, a multipoint location gas indication status interface is configured to enable one or more users to view and engage with one or more of a multipoint location gas indication aggregation interface component, an adjusted multipoint location gas indication aggregation interface component, a point location alert gas interface component, a point location service status interface component, a point location maintenance fault interface component, a point location instrument fault interface component, and/or a point location event interface component.

At block 1414, the multipoint location gas analysis system 200 includes means, such as the memory 201, processor 202, input/output circuitry 203, communications circuitry 204, and/or multipoint location gas analysis circuitry 205, or a combination thereof, to optionally generate a point location service status interface component, a point location maintenance fault interface component, a point location instrument fault interface component, or a point location event interface component. In some embodiments, a point location service status interface component may be configured to display text, colors, symbols, and/or the like representative of one or more service statuses associated with a multipoint location gas analysis system. In some embodiments, a point location maintenance fault interface component may be configured to display text, colors, symbols, and/or the like representative of one or more maintenance faults associated with a multipoint location gas analysis system. In some embodiments, a point location instrument fault interface component may be configured to display text, colors, symbols, and/or the like representative of one or more instrument faults associated with a multipoint location gas analysis system. In some embodiments, a point location event interface component may be configured to display text, colors, symbols, and/or the like representative of one or more events associated with a multipoint location gas analysis system.

At block 1416, the multipoint location gas analysis system 200 includes means, such as the memory 201, processor 202, input/output circuitry 203, communications circuitry 204, and/or multipoint location gas analysis circuitry 205, or a combination thereof, to optionally generate the point location alert gas data. In this regard, for example, a multipoint location gas analysis system may be configured to generate point location alert gas data by determining that a point location site is associated with a gas anomaly event. In some embodiments, a multipoint location gas analysis system may be configured to generate point location alert gas data by determining that a point location site is associated with a gas anomaly event using an optical analysis technique.

FIG. 14 thus illustrates a flowchart describing the operation of apparatuses, methods, systems, and computer program products according to example embodiments contemplated herein. It will be understood that each flowchart block, and combinations of flowchart blocks, may be implemented by various means, such as hardware, firmware, processor, circuitry, and/or other devices associated with execution of software including one or more computer program instructions. For example, one or more of the operations described above may be implemented by an apparatus executing computer program instructions. In this regard, the computer program instructions may be stored by a memory 201 of the multipoint location gas analysis system 200 and executed by a processor 202 of the multipoint location gas analysis system 200. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the functions specified in the flowchart blocks. The computer program instructions may also be loaded onto a computer or other programmable apparatus to cause a series of operations to be performed on the computer or other programmable apparatus to produce a computer-implemented process such that the instructions executed on the computer or other programmable apparatus provide operations for implementing the functions specified in the flowchart blocks.

The flowchart blocks support combinations of means for performing the specified functions and combinations of operations for performing the specified functions. It will be understood that one or more blocks of the flowcharts, and combinations of blocks in the flowcharts, can be implemented by special purpose hardware-based computer systems which perform the specified functions, or combinations of special purpose hardware with computer instructions.

Thus, particular embodiments of the subject matter have been described. While this specification contains many specific implementation details, these should not be construed as limitations on the scope of the present disclosure or of what may be claimed, but rather as description of features specific to particular embodiments of the present disclosure. Other embodiments are within the scope of the following claims. Certain features that are described herein in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results, unless described otherwise. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products. Any operational step shown in broken lines in one or more flow diagrams illustrated herein are optional for purposes of the depicted embodiment.

In some cases, the actions recited in the claims can be performed in a different order and still achieve desirable results. In addition, the processes depicted in the accompanying figures do not necessarily require the particular order shown, or sequential order, to achieve desirable results, unless described otherwise. In certain implementations, multitasking and parallel processing may be advantageous.

### CONCLUSION

Many modifications and other embodiments of the present disclosure set forth herein will come to mind to one skilled in the art to which this disclosure pertains having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An apparatus comprising at least one processor, and at least one memory including program code, the at least one memory and the program code configured to, with the at least one processor, cause the apparatus to at least:
access multipoint location gas indication data;
generate a multipoint location gas indication aggregation interface component based at least in part on the multipoint location gas indication data;
output the multipoint location gas indication aggregation interface component for rendering to a multipoint location gas indication status interface of a computing device associated with the multipoint location gas indication aggregation interface component;
generate a point location alert gas interface component based at least in part on point location alert gas data;
generate an adjusted multipoint location gas indication aggregation interface component; and
output the point location alert gas interface component and the adjusted multipoint location gas indication aggregation interface component for rendering to the multipoint location gas indication status interface of the computing device associated with the multipoint location gas indication aggregation interface component.

2. The apparatus of claim 1, wherein the multipoint location gas indication status interface comprises a first multipoint location gas indication status portion and a second multipoint location gas indication status portion.

3. The apparatus of claim 2, wherein the multipoint location gas indication aggregation interface component is configured to be rendered to the first multipoint location gas indication status portion and the second multipoint location gas indication status portion of the multipoint location gas indication status interface.

4. The apparatus of claim 2, wherein the adjusted multipoint location gas indication aggregation interface component is configured to be rendered to the first multipoint location gas indication status portion of the multipoint location gas indication status interface and the point location alert gas interface component to be rendered to the second multipoint location gas indication status portion of the multipoint location gas indication status interface.

5. The apparatus of claim 1, wherein the program code is further configured to, with the at least one processor, cause the apparatus to:
generate a point location service status interface component, a point location maintenance fault interface component, a point location instrument fault interface component, or a point location event interface component.

6. The apparatus of claim 1, wherein the program code is further configured to, with the at least one processor, cause the apparatus to:
generate the point location alert gas data, wherein generating the multipoint location gas indication data comprises:
determining that a point location site is associated with a gas anomaly event using an optical analysis technique.

7. The apparatus of claim 1, wherein the multipoint location gas indication data is indicative of at least one of a plurality of point location site identifiers, a plurality of point location unique identification identifiers, a plurality of point location gas type identifier, a plurality of point location gas concentration indicators, or a plurality of point location status indicators.

8. The apparatus of claim 1, wherein the multipoint location gas indication aggregation interface component comprises a plurality of point location gas indication interface components.

9. The apparatus of claim 8, wherein each of the plurality of point location gas indication interface components is configured to display at least one of a point location site interface component, a point location unique identification interface component, a point location gas type interface component, a point location gas concentration interface component, or a point location status interface component.

10. The apparatus of claim 9, wherein at least a portion of the point location alert gas interface component is associated with a deuteranopia type configuration, a protanopia type configuration, a tritanopia type configuration, or a monochromacy type configuration.

11. The apparatus of claim 1, wherein the point location alert gas interface component is configured to display at least one of a point location alert gas subsidiary interface component or a point location alert action gas interface component.

12. A method comprising:
accessing multipoint location gas indication data;
generating a multipoint location gas indication aggregation interface component based at least in part on the multipoint location gas indication data;
outputting the multipoint location gas indication aggregation interface component for rendering to a multipoint location gas indication status interface of a computing device associated with the multipoint location gas indication aggregation interface component;
generating a point location alert gas interface component based at least in part on point location alert gas data;
generating an adjusted multipoint location gas indication aggregation interface component; and
outputting the point location alert gas interface component and the adjusted multipoint location gas indication aggregation interface component for rendering to the multipoint location gas indication status interface of the computing device associated with the multipoint location gas indication aggregation interface component.

13. The method of claim 12, further comprising:
generating a point location service status interface component, a point location maintenance fault interface component, a point location instrument fault interface component, or a point location event interface component.

14. The method of claim 12, further comprising:
generating the point location alert gas data, wherein generating the multipoint location gas indication data comprises:
determining that a point location site is associated with a gas anomaly event using an optical analysis technique.

15. A computer program product comprising at least one non-transitory computer-readable storage medium having computer-readable program code portions stored therein, the computer-readable program code portions comprising an executable portion configured to:
access multipoint location gas indication data;
generate a multipoint location gas indication aggregation interface component based at least in part on the multipoint location gas indication data;
output the multipoint location gas indication aggregation interface component for rendering to a multipoint location gas indication status interface of a computing device associated with the multipoint location gas indication aggregation interface component;
generate a point location alert gas interface component based at least in part on point location alert gas data;
generate an adjusted multipoint location gas indication aggregation interface component; and
output the point location alert gas interface component and the adjusted multipoint location gas indication aggregation interface component for rendering to the multipoint location gas indication status interface of the computing device associated with the multipoint location gas indication aggregation interface component.
